(19) Europäisches Patentamt
European Patent Office
Office européen des brevets

(11) Publication number: 0 048 613
B1

(12) **EUROPEAN PATENT SPECIFICATION**

(45) Date of publication of patent specification: **23.01.85**

(51) Int. Cl.⁴: **C 07 H 15/22,** A 61 K 31/70 // C07D207/40

(21) Application number: **81304323.9**

(22) Date of filing: **21.09.81**

(54) **4-0-Substituted -2-deoxystreptamine aminoglycoside derivatives, their preparation and formulations containing them.**

(30) Priority: **22.09.80 US 189440**

(43) Date of publication of application: **31.03.82 Bulletin 82/13**

(45) Publication of the grant of the patent: **23.01.85 Bulletin 85/04**

(84) Designated Contracting States: **AT BE CH DE FR GB IT LI LU NL SE**

(56) References cited:
**THE JOURNAL OF ORGANIC CHEMISTRY, vol. 41, no. 12, June 11, 1976, S. O'CONNOR et al.: "Apramycin, a Unique Aminocyclitol Antibiotic", pages 2087-2092**
**JOURNAL OF THE AMERICAN CHEMICAL SOCIETY, vol. 100, no. 16, August 2, 1978, T.L. NAGABHUSHAN: "Interaction of Vicinal and Nonvicinal Amino-Hydroxy Group Pairs in Aminoglycoside-Aminocyclitol Antibiotics with Transition Metal Cations Selective N Protection" pages 2053-2054**

(73) Proprietor: **ELI LILLY AND COMPANY**
**307, East McCarty Street**
**Indianapolis Indiana 46285 (US)**

(72) Inventor: **Kirst, Herbert Andrew**
**1819 Madison Village Drive Apt. B-6**
**Indianapolis Indiana 46227 (US)**
Inventor: **Truedell, Brenda Alethia**
**3345, Washington Boulevard**
**Indianapolis Indiana 46205 (US)**

(74) Representative: **Hudson, Christopher Mark et al**
***Erl Wood Manor***
**Windlesham Surrey GU20 6PH (GB)**

(56) References cited:
**TETRAHEDRON LETTERS, no. 51, 1979, Pergamon Press Ltd., (GB) TSUTOMO-TSUCHIYA et al.: "1-N-Acylation of aminocyclitol antibiotics via zinc chelation and regiospecific N-Trifluoro-acetylation", pages 4951-4954**
**TETRAHEDRON LETTERS, vol. 22, 1981, Pergamon Press Ltd. G.B. H.A. KIRST et al.: "Control of site-specific substitution of aminoglycosides by transition metal cations", pages 295-298**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 048 613 B1

**Description**

The invention relates to novel 2′-N-acylated and 2′-N-alkylated derivatives of 4-O-substituted-2-deoxystreptamine amino-glycosides having antibacterial activity against a wide range of gram positive and gram negative bacteria. They are useful in pharmaceutical, veterinary and surface disinfectant formulations. These and related 2′-N-acylated and 2′-N-alkylated derivatives of related aminoglycosides are prepared with the help of a nickel salt.

Examples of 4-O-substituted 2-deoxystreptamine-containing aminoglycosides used in the process of the present invention include the antibiotics apramycin (see U.S. Patent Nos. 3,691,279, 3,853,709 and 3,876,767):

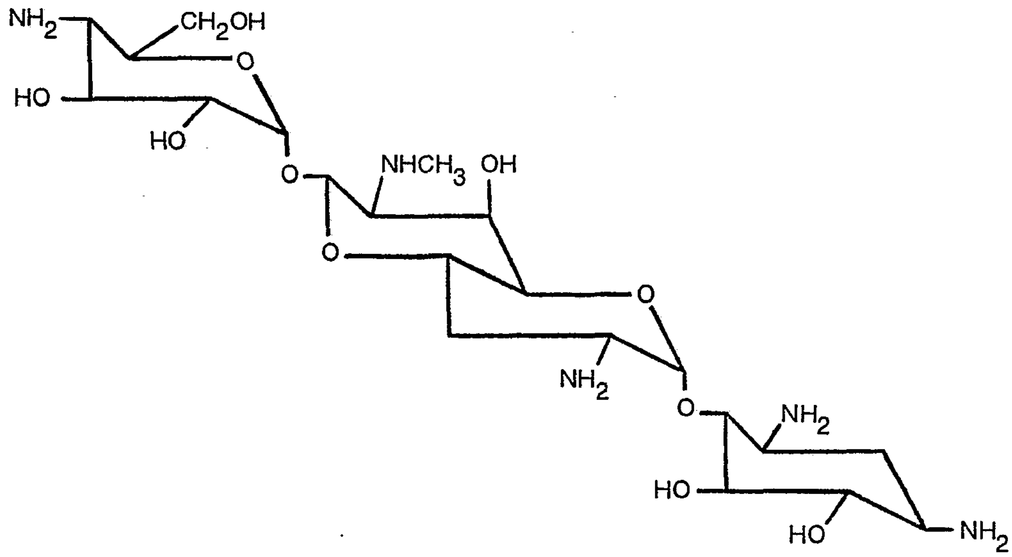

and nebramine:

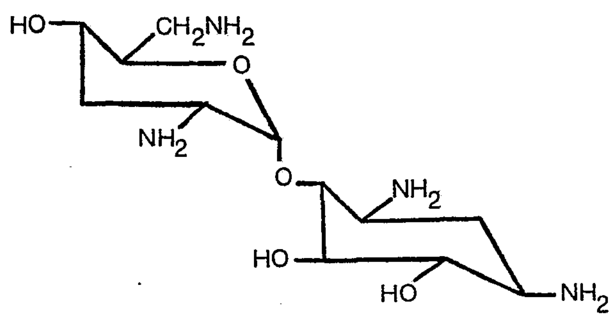

The nomenclature of the above formula of apramycin is simplified by the following numbering system in which the carbons of the 2-deoxystreptamine moiety are numbered 1 through 6, the carbons of the octose moiety are numbered with a single prime, 1′—8′ and the carbons of the hexose moiety are numbered with a double prime, 1″—6″.

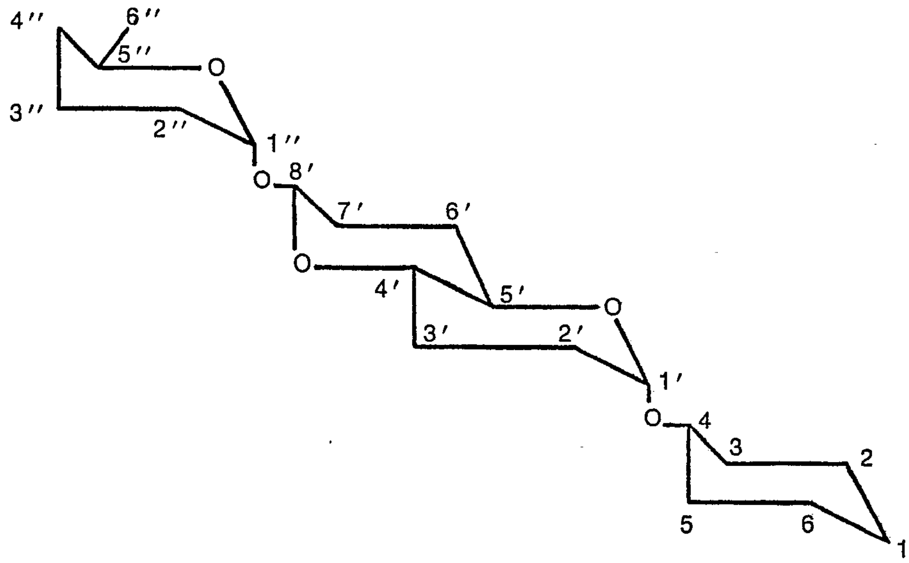

Analogously, the aprosanimide aminoglycosides referred to in this invention have the same numbering system as apramycin with respect to the octose and 2-deoxystreptamine moieties.

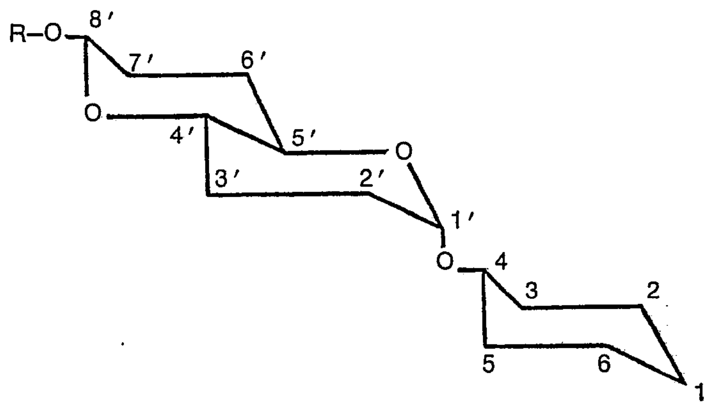

The numbering system for the pseudodisaccharides referred to in this invention, such as nebramine, has the carbons of the 2-deoxystreptamine moiety numbered 1—6 (the same as for apramycin and the aprosaminides) but the hexose carbons are numbered with a single prime, 1′—6′.

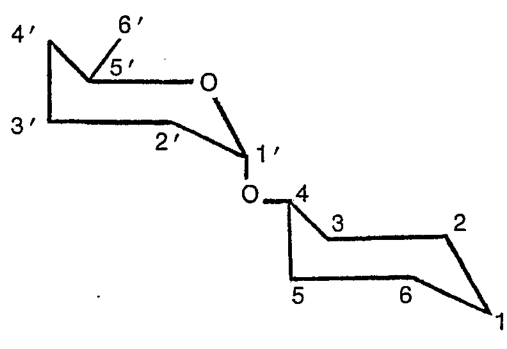

The prior art has not disclosed a direct acylation of the 2′-amino group of 2′-aminoglycoside antibiotics.

According to the present invention there is provided a 2′-N-monoacylated or 2′-N-monoalkylated derivative of a 4-O-substituted-2-deoxystreptamine aminoglycoside selected from the group consisting of;

apramycin,

α or β-($C_1$ to $C_6$ alkyl)aprosaminide,

α or β-($C_2$ to $C_6$ hydroxyalkyl)aprosaminide,

α or β-($C_2$ to $C_6$ aminoalkyl)aprosaminide,

α or β-($C_2$ to $C_6$ protected aminoalkyl)aprosaminide, α or β-($C_3$ to $C_6$ aminohydroxyalkyl)-

aprosaminide, $\alpha$ or $\beta$-($C_3$ to $C_6$ (protected amino)hydroxyalkyl)aprosaminide, or a pharmaceutically-acceptable acid addition salt thereof;

wherein the acyl or alkyl substituent group is selected from the group consisting of
$\gamma$-amino-$\alpha$-hydroxybutyryl, $\gamma$-protected amino-$\alpha$-hydroxybutyryl, $\beta$-amino-$\alpha$-hydroxypropionyl, $\beta$-protected amino-$\alpha$-hydroxypropionyl, glycyl, N-protected glycyl, 3-hydroxypyrrolidine-3-carbonyl, N-protected 3-hydroxypyrrolidine-3-carbonyl, 3-hydroxyazetidine-3-carbonyl, N-protected 3-hydroxyazetidine-3-carbonyl, 2-hydroxy-2-(azetidin-3-yl)acetyl, N-protected 2-hydroxy-2-(azetidin-3-yl)acetyl, 4-amino-2-hydroxybutyl, 3-amino-2-hydroxypropyl, 2-aminoethyl, 3-hydroxypyrrolidine-3-methylene, 3-hydroxyazetidine-3-methylene, 2-hydroxy-2-(azetidin-3-yl)ethyl, $C_2$ to $C_4$ alkyl, $C_3$ to $C_5$ allyl (i.e. allyl or alkyl allyl), benzyl and substituted benzyl.

This invention also provides formulations, pharmaceutical, veterinary, and adapted as a surface disinfectant, which comprise as an active ingredient a 2′-N-monoacylated or 2′-N-monoalkylated derivative of a 4-O-substituted-2-deoxystreptamine aminoglycoside as above described excepting compounds bearing protected amino or N-protected substituents associated with at least one carrier or diluent therefor, as appropriate to the formulation.

This invention also provides a process for preparing a 2′-N-monoacylated or 2′-N-monoalkylated derivative of a 4-O-substituted 2-deoxystreptamine aminoglycoside as before described or any one of the group lividamine, paromamine and the 6′-N-protected derivatives of nebramine, neamine, gentamine $C_1$, gentamine $C_{1a}$, gentamine $C_2$, gentamine $C_{2a}$, and gentamine $C_{2b}$, or a pharmaceutically-acceptable acid addition salt thereof which comprises the steps of:

(1) combining an aminoglycoside substrate selected from the group consisting of:
apramycin,
$\alpha$ or $\beta$-($C_1$ to $C_6$ alkyl)aprosaminide, $\alpha$ or $\beta$-($C_2$ to $C_6$ hydroxyalkyl)aprosaminide, $\alpha$ or $\beta$-($C_2$ to $C_6$ protected aminoalkyl)aprosaminide, $\alpha$ or $\beta$-($C_3$ to $C_6$ (protected amino)hydroxyalkyl)aprosaminide, lividamine, paromamine and the 6′-N-protected derivatives of nebramine, neamine, gentamine $C_1$, gentamine $C_{1a}$, gentamine $C_2$ gentamine $C_{2a}$, and gentamine $C_{2b}$;

with two to six molar equivalents of a nickel salt of the formula:

$$Ni(X)_2$$

in water;

(2) diluting the above aqueous mixture with between two and ten volumes of a polar organic solvent, and;

(3) adding between one and two molar equivalents of an acylating agent or between five and twenty molar equivalents of an alkylating agent to the above mixture of step (2) then carrying out the reaction at a temperature between 0°C and 40°C for a period between 0.5 and 24 hours for acylation or between 15 hours and 60 hours for alkylation, wherein:

X is $C_1$ to $C_{10}$ carboxylate;

The acylating agent used being a reactive derivative of an acyl moiety selected from the group consisting of $\gamma$-protected amino-$\alpha$-hydroxybutyryl, $\beta$-protected amino-$\alpha$-hydroxypropionyl, N-protected glycyl, N-protected 3-hydroxypyrrolidine-3-carbonyl, N-protected 3-hydroxyazetidine-3-carbonyl and N-protected 2-hydroxy-2-(azetidin-3-yl)acetyl; and

the alkylating agents used being $C_2$ to $C_4$ alkyl bromide, $C_2$ to $C_4$ alkyl iodide, $C_3$ to $C_5$ allyl bromide (i.e. allyl or alkyl allyl bromide), $C_3$ to $C_5$ allyl iodide (i.e. allyl or alkyl allyl iodide), benzyl bromide, benzyl iodide, substituted benzyl bromide and substituted benzyl iodide; and

(4) optionally, deprotecting protected amino or N-protected substituents;

(5) optionally, reducing the carbonyl portion of an acyl substituent with a reducing agent;

(6) recovering as a free base or as a pharmaceutically-acceptable acid addition salt thereof.

This invention also provides a 2′-N-monoacylated or a 2′-N-monoalkylated derivative of a 4-O-substituted 2-deoxystreptamine aminoglycoside as before described for use in treating warm-blooded animals having bacterial infections or for use as a surface disinfectant.

The process of this invention involves regio-selective acylation or alkylation of the 2′-amino group of 4-O-substituted -2-deoxystreptamine aminoglycoside substrates. The instant process is carried out by adding an acylating agent or alkylating agent to a mixture of the aminoglycoside substrate and a $C_1$ to $C_{10}$ carboxylate salt of nickel (II) in an aqueous polar organic solvent, and stirring the mixture for the appropriate time at a temperature between 0°C and 40°C.

The aminoglycoside substrates that are used in the process of this invention are selected from a group consisting of apramycin, $\alpha$ or $\beta$-($C_1$ to $C_6$ alkyl)aprosaminide, $\alpha$ or $\beta$-($C_2$ to $C_6$ hydroxyalkyl)-aprosaminide, $\alpha$ or $\beta$-($C_2$ to $C_6$ protected aminoalkyl)aprosaminide, and $\alpha$ or $\beta$-($C_3$ to $C_6$ (protected amino)hydroxyalkyl)aprosaminide, lividamine, paromamine and the 6′-N-protected derivatives of nebramine, neamine, gentamine $C_1$, gentamine $C_{1a}$, gentamine $C_2$, gentamine $C_{2a}$ and gentamine $C_{2b}$. The acylating agents for use in the present invention are reactive derivatives of acyl moieties selected from the group consisting of $\gamma$-protected amino-$\alpha$-hydroxybutyryl, $\beta$-protected amino-$\alpha$-hydroxypropionyl, N-protected glycyl, N-protected 3-hydroxypyrrolidine-3-carbonyl, N-protected 3-hydroxyazetidine-3-carbonyl and N-protected 2-hydroxy-2-(azetidin-3-yl)acetyl. The alkylating agents used in the instant process are $C_2$ to $C_4$ alkyl bromide, $C_2$ to $C_4$ alkyl iodide, $C_3$ to $C_5$ allyl bromide (i.e. allyl or alkyl allyl bromide), $C_3$ to $C_5$ allyl iodide (i.e. allyl or alkyl allyl iodide), benzyl bromide, benzyl iodide, substituted benzyl bromide and substituted benzyl iodide.

The compounds claimed in the present invention include the above-mentioned apramycin and the various α or β-aprosaminide compounds substituted on the 2′-amino group by the above-listed acyl or alkyl moieties. As isolated from the process of this invention, these acylated compounds have their 2′-N substituent in the protected amino or N-protected form. These derivatives in the protected amino and N-protected derivatives are intermediates to their respective deprotected amino and N-deprotected antibiotic compounds; which antibiotic compounds are also claimed in the instant application. Additionally, the deprotected amino acyl derivatives may undergo reduction of the carbonyl group to give a methylene group. These deprotected amino acyl derivative compounds and the N-deprotected compounds and all of the 2′-N alkyl derivatives, either in their free base form or their pharmaceutically acceptable acid addition salt form, are antibacterial agents and are also claimed compounds in the instant invention.

This invention is directed to the 2′-N-acylated and 2′-N-alkylated derivatives of apramycin, α or β-($C_1$ to $C_6$ alkyl)aprosaminide, α or β-($C_2$ to $C_6$ hydroxyalkyl)aprosaminide, α or β-($C_2$ to $C_6$ aminoalkyl)-aprosaminide, α or β-($C_2$ to $C_6$ protected aminoalkyl)aprosaminide, α or β-($C_3$ to $C_6$ aminohydroxy-alkyl)aprosaminide, α or β-($C_3$ to $C_6$ (protected amino) hydroxyalkyl)aprosaminide, and the pharmaceutically acceptable acid addition salts of these compounds. The acyl substituent on the 2′-amino group of the above compounds is selected from a group consisting of γ-amino-α-hydroxy-butyryl, γ-protected amino-α-hydroxybutyryl, β-amino-α-hydroxypropionyl, β-protected amino-α-hydroxypropionyl, glycyl, N-protected glycyl, 3-hydroxypyrrolidine -3-carbonyl, N-protected 3-hydroxypyrrolidine-3-carbonyl, 3-hydroxyazetidine-3-carbonyl, N-protected 3-hydroxyazetidine-3-carbonyl, 2-hydroxy-2-(azetidin-3-yl)acetyl and N-protected 2-hydroxy-2-(azetidin-3-yl)acetyl. Alkyl substituents on the 2′-amino group of the above compounds are selected from the group consisting of the reduced derivatives of the above deprotected-amino acyl substituents and N-deprotected acyl substituents, namely: 4-amino-2-hydroxybutyl, 3-amino-2-hydroxypropyl, 2-aminoethyl, 3-hydroxypyrrolidine-3-methylene, 3-hydroxyazetidine-3-methylene, 2-hydroxy-2-(azetidin-3-yl)ethyl, in addition to the alkyl substituents $C_2$ to $C_4$ alkyl, $C_3$ to $C_5$ allyl, benzyl and substituted benzyl.

In the above description of the invention, the term "$C_1$ to $C_6$ alkyl" means a branched or straight chain alkyl such as methyl, ethyl, n-propyl, i-propyl, n-butyl, i-butyl, sec-butyl, t-butyl, n-pentyl, α-methylpentyl, n-hexyl.

Similarly, by the term "$C_2$ to $C_6$ hydroxyalkyl", we mean from one to three hydroxyl groups bonded to any carbon atom of a $C_2$ to $C_6$ alkyl chain, provided that no more than one oxygen atom is bonded to any single carbon atom of the alkyl chain. Examples of the hydroxyalkyl chains include: 2-hydroxyethyl, 2-hydroxy-n-propyl, 3-hydroxy-n-propyl, 1,3-dihydroxy-2-propyl, 2,4-dihydroxy-n-butyl, 3-hydroxy-2-methylpropyl, 2,4,5-trihydroxy-n-pentyl, 2,4-dihydroxy-n-pentyl, 2-hydroxy-4-methylpentyl, 5-hydroxy-n-hexyl and 4,5,6-trihydroxy-n-hexyl groups.

By the term "$C_2$ to $C_6$ aminoalkyl", we mean from one to three amino groups bonded to any carbon atom of a $C_2$ to $C_6$ alkyl chain, provided that no more than one heteroatom is bonded to any single carbon atom of the alkyl chain. Examples of these aminoalkyl chains include: 2-aminoethyl, 2-amino-n-propyl, 3-amino-n-propyl, 1,3-diamino-2-propyl, 2,4-diamino-n-butyl, 3-amino-2-methylpropyl, 2,4,5-triamino-n-pentyl, 3-amino-n-pentyl, 2-amino-4-methylpentyl, 5,6-diamino-n-hexyl and 3-amino-n-hexyl groups.

By the term "$C_2$ to $C_6$ protected aminoalkyl", we mean from one to three protected amino groups bonded to any carbon atom of a $C_2$ to $C_6$ alkyl chain, provided that no more than one heteroatom is bonded to any single carbon atom of the alkyl chain. Examples of these protected aminoalkyl chains include the example compounds listed above for the $C_2$ to $C_6$ aminoalkyl chains wherein each of the amino groups are substituted by N-protecting groups.

By the term "$C_3$ to $C_6$ aminohydroxyalkyl", we mean a $C_3$ to $C_6$ alkyl chain substituted at any carbon atom by one amino group and one hydroxyl group, or by two amino groups and one hydroxyl group, or by one amino group and two hydroxyl groups, provided that no more than one heteroatom is bonded to any single carbon atom of the alkyl chain. Examples of these aminohydroxyalkyl chains include: 2-hydroxy-3-amino-n-propyl, 1-hydroxy-3-amino-2-propyl, 2-hydroxy-4-amino-n-butyl, 1-hydroxy-4-amino-2-butyl, 2-hydroxy-5-amino-n-pentyl, 4,5-diamino-3-hydroxy-n-pentyl, 3,5-dihydroxy-4-amino-n-pentyl, 2-amino-3-hydroxy-4-methylpentyl and 2-hydroxy-6-amino-n-hexyl groups.

By the term "$C_3$ to $C_6$ (protected amino)hydroxyalkyl", we mean a $C_3$ to $C_6$ alkyl chain substituted at any carbon atom by one protected amino group and one hydroxyl group, or by two protected amino groups and one hydroxyl group, or by one protected amino group and two hydroxyl groups, provided that no more than one heteroatom is bonded to any single carbon atom of the alkyl chain. Examples of these (protected amino)hydroxyalkyl chains include the examples listed above for the $C_3$ to $C_6$ amino-hydroxyalkyl chains wherein each of the amino groups are substituted by N-protecting groups.

The terms "protected amino" and "N-protected" used in the above description refer to an amino

group substituted with one of the commonly employed amino blocking groups that are compatible with the present reaction conditions. Representative suitable amino-blocking groups for the purpose of this invention are described by J. W. Barton in "Protective Groups in Organic Chemistry", J. F. W. McOmie, Ed., Plenum Press, New York, N.Y., 1973, Chapter 2 and T. W. Greene "Protective Groups in Organic Synthesis", John Wiley and Sons, New York, N.Y., 1981, Chapter 7. Preferred amino blocking groups are the various alkoxycarbonyl and aryloxycarbonyl groups, with the most preferred amino blocking group being benzyloxycarbonyl.

By the term "pharmaceutically acceptable acid addition salts" we mean those formed by standard acid-base reactions between the appropriate aminoglycoside and organic or inorganic acids. Such acids include hydrochloric, sulfuric, phosphoric, acetic, succinic, citric, latic, maleic, fumaric, palmitic, cholic, pamoic, mucic, D-glutamic, *d*-camphoric, glutaric, glycolic, phthalic, tartaric, lauric, stearic, salicylic, methanesulfonic, benzenesulfonic, sorbic, picric, benzoic and cinnamic acids.

By the term "$C_2$ to $C_4$ alkyl", we mean ethyl, n-propyl and n-butyl.

By the term "$C_3$ to $C_5$ allyl" we mean allyl, 2-methylallyl, 3,3-dimethylallyl, 3-methylallyl, and 2,3-dimethylallyl. Those skilled in the art will recognize that the latter two allyl substituents can exist in either the E or Z geometric isomers. In the present invention, it is understood that the allyl compounds can exist in the pure E or Z form, or as a mixture of the two isomers.

By the term "substituted benzyl" we mean a benzyl moiety where the phenyl ring is substituted with one substituent selected from the group consisting of fluoro, chloro, bromo, iodo, methyl, methoxy and trifluoromethyl. This substituent can be in either the ortho, meta or para positions. Examples of these substituted benzyl groups include para-chlorobenzyl, para-bromobenzyl, paraiodobenzyl, para-methylbenzyl, para-methoxybenzyl, para-trifluoromethylbenzyl, meta-fluorobenzyl, meta-bromobenzyl, meta-methylbenzyl, meta-methoxybenzyl, meta-chlorobenzyl, ortho-chlorobenzyl, ortho-iodobenzyl, ortho-methylbenzyl, ortho-methoxybenzyl and ortho-bromobenzyl.

Those skilled in the art will recognize that all of the above listed acyl substituents except glycyl and N-protected glycyl, the corresponding reduced acyl substituents, namely: 4-amino-2-hydroxybutyl, 3-amino-2-hydroxypropyl, 3-hydroxypyrrolidine-3-methylene, 3-hydroxyazetidine-3-methylene, 2-hydroxy-2-(azetidin-3-yl)ethyl, as well as many of the substituents described by the terms "$C_1$ to $C_6$ alkyl", "$C_2$ to $C_6$ hydroxyalkyl", "$C_2$ to $C_6$ aminoalkyl", "$C_2$ to $C_6$ protected aminoalkyl", "$C_3$ to $C_6$ aminohydroxyalkyl" and "$C_3$ to $C_6$ (protected amino)hydroxyalkyl" can exist as optical isomers. These terms refer to either the individual R or S isomers or to equal or unequal mixtures of the R and S isomers at each of the asymmetric carbon atoms for the purposes of this invention.

The process of making the above 2′-N-acylated and 2′-N-alkylated compounds is the other aspect of this invention. Specifically, this process substantially selectively acylates or alkylates the 2-amino group of the following substrates:

apramycin, $\alpha$ or $\beta$-($C_1$ to $C_6$ alkyl)aprosaminide, $\alpha$ or $\beta$-($C_2$ to $C_6$ hydroxyalkyl)aprosaminide, $\alpha$ or $\beta$-($C_2$ to $C_6$ protected aminoalkyl)aprosaminide, $\alpha$ or $\beta$-($C_3$ to $C_6$ (protected amino)hydroxyalkyl)-aprosaminide, lividamine, paromamine, and the 6′-N-protected derivatives of nebramine, neamine, gentamine $C_1$, gentamine $C_{1a}$, gentamine $C_2$, gentamine $C_{2a}$, and gentamine $C_{2b}$, by

(1) combining the above aminoglycoside with between two and six molar equivalents of a nickel salt of the formula:

$$Ni(X)_2$$

in water;

(2) diluting the above mixture with between about two and ten volumes of a polar organic solvent; and

(3) adding between one and two molar equivalents of an acylating agent or between five and twenty molar equivalents of an alkylating agent to the above dilution of step (2) then carrying out the reaction at a temperature between 0°C and 40°C for a period between 0.5 and 24 hours for acylation, and between 15 hours and 60 hours for alkylation.

The terms "$C_1$ to $C_6$ alkyl", "$C_2$ to $C_6$ hydroxyalkyl", "$C_2$ to $C_6$ protected aminoalkyl", "$C_3$ to $C_6$ (protected amino)hydroxyalkyl", "protected amino" and "...N-protected..."are as defined above for the compounds of the instant invention.

Three of the above-listed aminoglycoside substrates for the process of this invention, namely apramycin, lividamine and paromamine, are known compounds and therefore their synthesis or isolation can be found in the literature.

The pseudodisaccharides nebramine, neamine, gentamine $C_1$, gentamine $C_{1a}$, gentamine $C_2$, gentamine $C_{2a}$, and gentamine $C_{2b}$ are also known compounds and therefore their synthesis or isolation can also be found in the literature. These pseudodisaccharides can be converted to their 6′-N-protected derivatives which are required as substrates for the instant process by methods analogous to the procedure given in Example 4, Part A of the Experimental Section.

The various substituted $\alpha$ or $\beta$ aprosaminide substrates listed above can be made by methods analogous to the procedure described by S. O'Connor, *et al.,* Journal of Organic Chemistry, *41*, 2087 (1976).

The acylating agents used in the instant process are also known in the art. Additional examples

for converting the known carboxylic acid precursors into suitable acylating agents for the process of this invention are given in Preparations 1 and 2 in the Experimental Section.

As with the compounds claimed in the present invention, it will be recognized by those skilled in the art that certain of the acylating agents, except for the reactive derivative of N-protected glycyl, certain $C_1$ to $C_6$ alkyl chains and certain substituted $C_2$ to $C_6$ alkyl chans and $C_3$ to $C_6$ alkyl chains of the $\alpha$ or $\beta$ substituted aprosaminide substrates can exist as optical isomers. For the purposes of the process of this invention, these moieties can be used as their pure R or S configurations or as mixtures of equal or unequal proportions of these two configurations at each of the asymmetric carbon atoms.

In the above description of the process of the instant invention, the formula:

$$Ni(X)_2$$

refers to a nickel salt wherein the nickel is in the formal (II) oxidation state, and X refers to a $C_1$ to $C_{10}$ carboxylate ligand. The term "$C_1$ to $C_{10}$ carboxylate" includes straight or branched chain aliphatic carboxylates, as well as aromatic carboxylates, with acetate being the preferred carboxylate. Exemplary nickel salts used in the instant process include:

$Ni(acetate)_2$
$Ni(propionate)_2$
$Ni(hexanoate)_2$
$Ni(2\text{-ethylhexanoate})_2$
$Ni(benzoate)_2$
$Ni(octanoate)_2$
$Ni(neodecanoate)_2$.

Although not explicitly depicted in the above formula and examples, the nickel salts used in the process of this invention may be either anhydrous or hydrated.

The term "acylating agent", as used in the above description of the instant process, means a reactive derivative of a carboxylic acid which is capable in the instant process of coupling the acid to an amino group by an amide linkage. The carboxylic acids that are converted into reactive derivatives for use as acylating agents in the process of this invention are selected from a group consisting of $\gamma$-protected amino-$\alpha$-hydroxybutyric acid, $\beta$-protected amino-$\alpha$-hydroxypropionic acid, N-protected glycine, N-protected 3-hydroxypyrrolidine-3-carboxylic acid, N-protected 3-hydroxyazetidine-3-carboxylic acid, and N-protected 2-hydroxy-2-(azetidin-3-yl)acetic acid. Examples of reactive derivatives of a carboxyl group are an acid halide, an acid anhydride, an activated ester, an acid azide. Preferred reactive derivatives are acid anhydrides and activated esters, with the more preferred reactive derivatives being acid anhydrides and the activated esters formed with N-hydroxysuccinimide and N-hydroxyphthalimide.

After the 2′-(N-protected and amino-protected) acyl derivative compounds have been prepared by the instant process, they can be converted to their corresponding deprotected acyl derivatives by procedures well known in the art.

Furthermore, the above deprotected amino or deprotected nitrogen acyl derivative compounds may have their carbonyl groups reduced to methylene groups with diborane or lithium aluminum hydride; these newly formed alkyl derivative compounds still retain biological activity. Methods for reducing the carbonyl groups are well known in the art, and include methods such as the ones described by K. Igarashi, *et al.* in U.S. Patent 4,201,774. An additional reference for the above reduction procedure is K. Richardson *et al.,* J. Antibiotics, *30*, 843, (1977).

The alkylating agents used in the instant process are $C_2$ to $C_4$ alkyl bromide, $C_2$ to $C_4$ alkyl iodide, $C_3$ to $C_5$ allyl bromide, $C_3$ to $C_5$ allyl iodide, benzyl bromide, benzyl iodide, substituted benzyl bromide and substituted benzyl iodide. The preferred alkylating agents are ethyl iodide, n-butyl bromide, n-butyl iodide, allyl bromide, allyl iodide, and benzyl bromide.

It should be noted that the instant process will result in the 2′, 6′-di-N-acylation of nebramine, neamine, gentamine $C_1$, gentamine $C_{1a}$, gentamine $C_2$, gentamine $C_{2a}$, and gentamine $C_{2b}$ when the 6′-amino group of these substrates is not protected. Therefore, in order to acylate the above pseudodisaccharide substrates on the 2′-amino group only, it is necessary to block the 6′-amino group with a suitable amino protecting group as defined above. This protecting group can then be removed after the 2′-amino group is substituted to yield the desired aminoglycoside derivative. An example of a 6′-N protection→2′-N acylation→6′-N deprotection procedure is given in Example 4 of the Experimental Section.

The first step of the instant process involves forming the nickel salt:aminoglycoside complex. This step is accomplished by mixing the aminoglycoside substrate compound and the nickel salt in water. Thorough mixing of these two components in the water is crucial and can be accomplished by employing methods such as sonication, vigorous stirring and mixing, moderate warming, etc. An aqueous solution is usually obtained; if not, a thoroughly mixed or dispersed suspension is sufficient.

Between about a 2:1 to about a 6:1 molar ratio of nickel salt:aminoglycoside substrate can be used, with the usual ratio employed being about 4:1.

The second step of the instant process entails diluting the above mixture with a polar, organic solvent. By the term "polar, organic solvent" we mean a solvent such as formamide, dimethylformamide, dimethylacetamide, dimethyl sulfoxide, hexamethylphosphoramide, t-butanol, tetrahydrofuran, acetonitrile, acetone, sulfolane, tetramethylurea, 2-pyrrolidinone, N-methylpyrrolidinone and dioxane or a mixture of any of the above solvents, with the preferred solvent for acylation being dimethylformamide. The preferred "polar, organic solvent" for alkylation is also dimethylformamide. Between two and ten volumes of polar, organic solvent per volume of water can be used, with the usual amount employed being about 5 volumes of organic solvent per volume of water. The resultant mixture is usually stirred for about five minutes to about one hour before proceeding to the next step. The mixture can be either heterogeneous or homogeneous at this point.

The final step of the instant process involves adding the appropriate acylating or alkylating agent to the mixture of step 2 of the process. The amount of acylating agent that can be used ranges from a 1:1 to a 2:1 ratio of acylating agent:aminoglycoside with about a 1.5:1 ratio being typical. A ratio range of 5:1 to 20:1 of alkylating agent:aminoglycoside can be used, with the usual ratio employed being about 10:1.

The time required to complete the acylation in the final step is not critical. The acylation reaction can be stirred for a period ranging between 0.5 and 24 hours, with the usual time being about 15 hours. The alkylation reaction requires longer periods of time, encompassing a range of fifteen hours to sixty hours.

The temperature at which the instant process is carried out can range from between 0°C to 40°C. Typically the reaction is run between 20°C to 25°C.

Once the acylation reaction of the final step is completed, recognized isolation procedures may be utilized to obtain the desired purified product. One such procedure involves column chromatography on either silica gel or Chelex 100 (Registered Trade Mark) resin (BioRad Labs, 2200 Wright Ave., Richmond, Ca. 94804) to adsorb the nickel salt from and separate the desired product from the resultant product mixture obtained from the instant process. To carry out this procedure, the solvent is evaporated *in vacuo* from the product mixture. The resultant residue is dissolved or suspended in an appropriate solvent and then loaded onto a column packed with the same solvent, using either Chelex 100 resin or silica gel as the stationary phase. An example of an appropriate solvent for use with the Chelex 100 resin is aqueous ammonium hydroxide. An example of an appropriate solvent for use with the silica gel is a 30:10:1 volume ratio of dichloromethane:methanol:ammonium hydroxide. The column is then eluted by common procedures (gradient or stepwise). The product can be chromatographed again on a second column of ion-exchange resin (e.g., Bio-Rex 70) or silica gel if necessary.

Chelex 100 is a styrene divinylbenzene copolymer functionalized with iminodiacetate ions which act as chelating ligands for binding metal ions.

Another isolation procedure removes Ni(II) ions by precipitation as the insoluble sulfide salts followed by ion-exchange chromatography to separate the desired products. Specifically, this procedure involves a) evaporating the solvent from the product mixture, dissolving the resultant residue in dilute acid (e.g., 0.1N HCl) and bubbling hydrogen sulfide gas through this mixture until precipitation of the nickel ion is complete and b) chromatographing the filtered solution from step a) on an ion-exchange resin such as Bio-Rex 70 (BioRad Labs, supra) using an ammonium hydroxide solution or gradient to elute the desired product.

Bio-Rex 70 (Registered Trade Mark) is a weakly acidic cation exchange resin containing carboxylic acid groups on a macro-reticular acrylic polymer lattice. Similar useful ion-exchange resins are Amberlite CG-50 and Amberlite IRC-50 (Registered Trade Marks) (Rohm and Haas Co., Philadelphia, Pa.).

The 2′-N-acylated and 2′-N-alkylated aminoglycosides made by the process of this invention, some of which having the 2′-N substituent in the protected amino or N-protected form, and in addition some of the various pseudodisaccharides having the 6′-amino group protected, are intermediates to their respective deprotected amino and N-deprotected compounds. These 2′-(protected amino and N-protected)acyl derivatives can be converted to their corresponding deprotected forms according to procedures well recognized in the art. These deprotected compounds, either in their free base form or their pharmaceutically acceptable acid addition salt form, are useful for treating infections in warm-blooded animals caused by gram-positive and gram-negative bacteria. These compounds can be administered parenterally, in the free base form or in the pharmaceutically acceptable acid addition salt form, using pharmaceutically acceptable formulations known in the art. These compounds can also be administered as veterinary compositions, such as, for example, in the feed or drinking water of farm animals to treat infections such as colibacillosis or swine dysentery.

Alternatively, these compounds can be used as a surface disinfectant. Solutions containing as little as 0.01 percent by weight of the antibiotic are effective for disinfecting purposes. Such solutions, preferably also containing a detergent or other cleansing agent, are useful for disinfecting objects such as glassware, dental and surgical instruments, and surfaces such as walls, floors, and tables in areas

where maintenance of sterile conditions is important, i.e. hospitals, food-preparation areas, and the like.

The antibacterial activity of the deprotected compounds of this invention is illustrated by the following *in vitro* and *in vivo* test data obtained with representative compounds. In Table I, the minimum inhibitory concentration (MIC) for representative compounds against a wide range of gram-positive and gram-negative bacteria is presented. The MIC values were obtained by the standard agar dilution test.

TABLE I

Antibiotic activity of 2′-N-substituted derivatives of apramycin and nebramine
vs.
gram-positive and gram-negative bacteria

| Test organism* | Test compound[1] Minimum inhibitory concentration (mcg/ml) | | | | |
|---|---|---|---|---|---|
| | 1 | 2 | 3 | 4 | 5 |
| Staphylococcus aureus X1.1 | 16 | 16 | 16 | 4 | 32 |
| Staphylococcus aureus V41 | 32 | 32 | 64 | 8 | 64 |
| Staphylococcus aureus X400 | 64 | 64 | 128 | 16 | 64 |
| Staphylococcus aureus S13E | 16 | 16 | 32 | 8 | 32 |
| Staphylococcus epidermidis EPI1 | 16 | 16 | 16 | 8 | 64 |
| Staphylococcus epidermidis EPI2 | 4 | 8 | 8 | 2 | 32 |
| Streptococcus pyogenes C203 | 32 | 32 | 64 | 16 | 128 |
| Streptococcus pneumoniae Park I | 32 | 32 | 128 | 16 | 128 |
| Streptococcus group D X66 | >128 | >128 | >128 | 128 | >128 |
| Streptococcus group D 9960 | >128 | >128 | >128 | >128 | >128 |
| Hemophilus influenzae C.L. | 64 | 32 | 16 | 16 | 64 |
| Hemophilus influenzae 76 | 32 | 32 | 32 | 16 | 64 |
| Shigella sonnei N9 | 32 | 32 | 16 | 8 | 64 |
| Escherichia coli N10 | 16 | 16 | 64 | 8 | 128 |
| Escherichia coli EC14 | 32 | 32 | 32 | 8 | >128 |
| Escherichia coli TEM | 8 | 8 | 8 | 2 | 32 |
| Klebsiella pneumoniae X26 | 8 | 8 | 8 | 4 | 64 |
| Klebsiella pneumoniae KAE | 16 | 32 | 16 | 8 | 64 |
| Enterobacter aerogenes X68 | 16 | 16 | 16 | 8 | 64 |
| Enterobacter aerogenes C32 | 16 | 16 | 16 | 8 | 64 |
| Enterobacter aerogenes EB17 | 16 | 32 | 16 | 8 | 64 |
| Enterobacter cloacae EB5 | 32 | 32 | 32 | 16 | 128 |
| Enterobacter cloacae 265A | 64 | 64 | 32 | 32 | >128 |
| Salmonella heidelberg X514 | 32 | 32 | 32 | 16 | 128 |

TABLE 1 (Continued)
Antibiotic activity of 2′-N-substituted derivatives of apramycin and nebramine
vs.
gram-positive and gram-negative bacteria

| Test organism* | Test compound[1] Minimum inhibitory concentration (mcg/ml) | | | | |
|---|---|---|---|---|---|
| | 1 | 2 | 3 | 4 | 5 |
| Salmonella typhimurium 1335 | 16 | 32 | 64 | 8 | 128 |
| Pseudomonas aeruginosa X528 | 128 | 128 | 128 | 64 | >128 |
| Pseudomonas aeruginosa X239 | 64 | 128 | 64 | 64 | 128 |
| Pseudomonas aeruginosa Ps18 | 128 | 128 | 128 | 32 | 128 |
| Serratia marcescens X99 | 16 | 16 | 16 | 8 | 128 |
| Serratia marcescens SE3 | 32 | 32 | 32 | 16 | >128 |
| Proteus morganii PR15 | 128 | 128 | 128 | 32 | 64 |
| Proteus inconstans PR33 | 16 | 32 | 32 | 8 | 64 |
| Proteus rettgeri PR7 | 8 | 16 | 32 | 8 | 32 |
| Proteus rettgeri C24 | 16 | 16 | 16 | 8 | 64 |
| Citrobacter freundii CF17 | 32 | 32 | 64 | 16 | 64 |
| Bordetella bronchiseptica 16 | 128 | 128 | 128 | 32 | >128 |

*Numerals and letters following the names of test microorganisms refer to the strains.
[1]Test compounds numbered 1—5 are as follows:
1=2′-N-(S-γ-Amino-α-hydroxybutyryl)apramycin
2=2′-N-(RS-γ-Amino-α-hydroxybutyryl)apramycin (racemic side chain)
3=2′-N-Glycylapramycin
4=2′-N-ethylapramycin
5=2′-N-(S-γ-Amino-α-hydroxybutyryl)nebramine.

Table II illustrates the effective dose ($ED_{50}$, effective dose to protect 50 percent of the test animals) for representative compounds of this invention against an experimental bacterial infection in mice.

TABLE II
Subcutaneous therapy of *Staphylococcus aureus* 3055 infections in mice

| Test compound | Effective dose ($ED_{50}$)* |
|---|---|
| 2′-N-(S-γ-Amino-α-hydroxybutyryl)apramycin | 6.5[1] |
| 2′-N-(RS-γ-Amino-α-hydroxybutyryl)apramycin[4] | 21.8[2] |
| 2′-N-glycylapramycin · HCl | 30[3] |
| 2′-N-ethylapramycin | <4.35[1] |
| 2′-N-(S-γ-Amino-α-hydroxybutyryl)nebramine | >70[3] |

*mg/kg×2 subcutaneous doses at 1 and 5 hours post infection.
[1]Mice were challenged with 68.1 $LD_{50}$'s.
[2]Mice were challenged with 331 $LD_{50}$'s.
[3]Mice were challenged with 1794 $LD_{50}$'s.
[4]Mixture of diastereomers.

The $ED_{50}$ values were determined as described by W. E. Wick *et al.,* Journal of Bacteriology, *81* [No. 2], 233—235 (1961).

The following Examples (1—8) are provided to further illustrate this invention. Preparations 1 and 2 provide examples of methods for synthesizing activated esters used in the process of the present invention. It is not intended that this invention be limited in scope by reason of any of the preparations or examples. In the following preparations and examples, carbon-13 nuclear magnetic resonance spectra, field desorption mass spectra and optical rotations are abbreviated c.m.r., f.d.m.s., and o.r., respectively. The nuclear magnetic resonance spectra were obtained on either a Varian Associates FT-80 Spectrometer or a JEOL JNM-PS-100 Spectrometer using dioxane as the internal standard. The chemical shifts are expressed in δ values (parts per million downfield from tetramethylsilane. The field desorption mass spectra were taken on a Varian-MAT 731 Spectrometer using carbon dendrite emitters. Optical rotations were measured on a Perkin Elmer Model 241 Polarimeter. Thin layer chromatography was carried out on E. Merck silica gel plates.

Preparation 1

Preparation of N-(S-γ-benzyloxycarbonylamino-α-hydroxybutyryloxy)succinimide

S-γ-Benzyloxycarbonylamino-α-hydroxybutyric acid (10.1 g, 40 mmol) and N-hydroxysuccinimide (4.60 g, 40 mmol) were dissolved in ethyl acetate (220 ml) and cooled in an ice bath. N,N′-Dicyclohexylcarbodiimide (8.25 g, 40 mmol) was added and the reaction mixture was stirred overnight. The resulting white precipitate was filtered and the filtrate was evaporated to dryness under reduced pressure. The residue was triturated with pentane (200 ml). The resultant white solid was filtered, washed with pentane and air-dried to yield 9.37 g (67% of product. Crystallization from ethyl acetate yielded purified material, m.p. 108°C.

Preparation 2

Preparation of N-(N-benzyloxycarbonylglycyloxy)succinimide

N-Benzyloxycarbonylglycine (10.9 g, 52.2 mmol) and N-hydroxysuccinimide (6.0 g, 52.2 mmol), were dissolved in dioxane (100 ml) and cooled in a cold water bath. N,N′-Dicyclohexylcarbodiimide (10.8 g, 52.2 mmol) was added and the mixture was stirred overnight. The white precipitate which formed was filtered, washed and air-dried (11.8 g, 100% of dicyclohexylurea). The filtrate was evaporated to dryness under reduced pressure, and the residual solid (16.2 g) was crystallized from ethyl acetate (50 ml) to give 8.4 g (53%) of crystalline N-(N-benzyloxycarbonylglycyloxy)succinimide, m.p. 110.5—112°C.

Example 1

Preparation of 2′-N-(S-γ-amino-α-hydroxybutyryl)apramycin

Apramycin (1.08 g, 2.0 mmol) and nickel(II) acetate tetrahydrate (1.99 g, 8 mmol) were dissolved in water (20 ml) and this solution was diluted with dimethylformamide (100 ml). The resultant opaque, blue-green mixture was stirred for one hour at ambient temperature and then treated with N-(S-γ-benzyloxycarbonylamino-α-hydroxybutyryloxy)succinimide (1.05 g, 3 mmol); a homogeneous green solution formed after about thirty minutes. After stirring overnight at ambient temperature, the solvent was evaporated under reduced pressure. The resultant residue was dissolved in a 30:15:2.5 mixture of dichloromethane:methanol:concentrated ammonium hydroxide and was loaded onto a silica gel column (Grade 950, 200 g) prewashed with the same solvent mixture. The column was successively

eluted with three different mixtures of dichloromethane:methanol:concentrated ammonium hydroxide (30:15:2.5, 6:6:1, 3:3:1). The fractions containing the desired product were combined, the solvent was evaporated and the resultant colorless glassy residue was dissolved in water and was lyophilized to give 2′-N-(S-γ-benzyloxycarbonylamino-α-hydroxybutyryl)apramycin (0.60 g, 39% yield.

The above 2′-N-(S-γ-benzyloxycarbonylamino-α-hydroxybutyryl)apramycin (550 mg) was dissolved in a solution of water (30 ml), dioxane (10 ml) and glacial acetic acid (1 ml). 5% palladium on carbon (135 mg) was added and the mixture was hydrogenated at 45 p.s.i. for 24 hours at room temperature. The palladium catalyst was removed by filtration and the filtrate was evaporated to dryness. The residual glassy product was dissolved in water and loaded onto a column of Bio-Rex 70 (290 ml, ammonium hydroxide cycle, pH 10.0). This column was eluted with a linear gradient of 0.005N ammonium hydroxide (1 l) and 0.5N ammonium hydroxide (1 l) followed by continued elution with 0.5N ammonium hydroxide. The fractions containing the desired product were combined, concentrated and lyophilized to yield 2′-N-(S-γ-amino-α-hydroxybutyryl)apramycin (383 mg, 84% yield): c.m.r. ($D_2O$—$H_2O$, pH≥10) δ 29.7, 32.9, 33.2, 36.3, 37.7, 48.8, 50.4, 51.0, 53.1, 61.6, 62.2, 66.1, 67.5, 70,5, 71.2, 71.7, 73.4, 74.2, 76.6, 78.3, 87.4, 95.4, 96.5, 98.5, 175.9; (at pH≤2) δ 29.0, 29.2, 31.1, 31.7, 37.6, 47.9, 50.0, 50.6, 53.0, 60.4, 61.2, 63.7, 67.7, 69.0, 70.1, 70.3, 70.6, 71.2, 73.4, 76.1, 78.8, 93.7, 95.3, 97.5, 175.5; f.d.m.s. m/e 623 (P+ 1—18); thin layer chromatography, $R_f$=0.14 using a 3:1:2 volume ratio of methanol:methylene chloride:concentrated ammonium hydroxide.

Example 2

Preparation of 2′-N-(RS-γ-amino-α-hydroxybutyryl)apramycin

The same procedure was followed as described in Example 1, except the acylating agent employed was N-(RS-γ-benzoyloxycarbonylamino-α-hydroxybutyryloxy)succinimide (racemic mixture). The product obtained from this reaction, 2′-N-(RS-γ-amino-α-hydroxybutyryl)apramycin, had the following physical properties: c.m.r. ($D_2O$—$H_2O$, pH <3) δ 29.3 (2x), 31.4, 31.9, 37.8, 48.0, 50.2, 51.0, 53.4, 60.6, 61.4, 64.0, 67.7, 69.2, 70.2, 70.3, 70.6, 71.3, 73.6, 76.2, (78.8 and 79.1 -doublet), 93.7, 95.3, 97.4, 175.6; f.d.m.s. m/e 641 (p+1); o.r. $[\alpha]_D^{25°}$=+124.7° ($H_2O$, c=10 mg/ml).

Example 3

Preparation of 2′-N-Glycylapramycin

Apramycin (2.16 g, 4 mmol) and nickel (II) acetate tetrahydrate (4.0 g, 16 mmol) were dissolved in water (40 ml) and the resultant solution was diluted with dimethylformamide (200 ml). To this mixture was added N-(N-benzoyloxycarbonylglycyloxy)succinimide (1.84 g, 6 mmol). After stirring overnight at ambient temperature, solvent was evaporated under reduced pressure. The residue was dissolved in 0.1 N hydrochloric acid (250 ml) and a slow steam of gaseous hydrogen sulfide was bubbled into the mixture for one hour. After stirring overnight, the precipitate was filtered and the filtrate was evaporated to dryness under reduced pressure. The resultant residue was suspended in 1 N sodium hydroxide (35 ml) and was loaded on a column of 300 ml Bio-Rex 70 (ammonium hydroxide cycle, pH 10, pre-eluted with 0.01 N ammonium hydroxide). The column was eluted first with 0.01 N ammonium hydroxide (400 ml) and then with a linear gradient of 0.01 N ammonium hydroxide (2 l) and 0.05 N ammonium hydroxide (2 l). Fractions containing the desired product were located by thin layer chromatography, combined and lyophilized to yield 2′-N-(N-benzyloxycarbonylglycyl)apramycin (1.35 g, 46% yield).

The above 2′-N-(N-benzoyloxycarbonylglycyl)apramycin was dissolved in a mixture of ethanol (100 ml), water (10 ml) and 1 N hydrochloric acid (10 ml). 5% palladium on charcoal (3 g) was added. This mixture was hydrogenated at 60 p.s.i. at ambient temperature overnight. The palladium catalyst was removed by filtration and the filtrate was placed in the refrigerator overnight. The desired crystalline 2′-N-glycylapramycin pentahydrochloride (1.01 g, 78% yield) precipitated from solution and was collected by filtration, washed with ethanol and air-dried: c.m.r. ($D_2O$—$H_2O$, pH ≥10) δ 29.7, 32.8, 35.1, 44.2, 48.4, 50.3, 51.1, 53.1, 61.6, 62.1, 66.1, 67.4, 71.2, 71.7, 73.4, 74.2, 76.6, 77.4, 87.0, 95.4, 96.3, 98.5, 174.5; f.d.m.s. m/e 597 (P+1); o.r. $[\alpha]_D^{25°}$=+127.3° ($H_2O$, C=8.3 mg/ml); thin layer chromatography, $R_f$=0.32 using a 3:1:2 volume ratio of methanol:methylene chloride:concentrated ammonium hydroxide.

Example 4

A. Preparation of 6′-N-Benzyloxycarbonylnebramine

Nebramine (15.3 g, 50 mmol) was dissolved in water (400 ml) and tetrahydrofuran (THF) (150 ml). A solution of benzyloxycarbonylimidazole (11.6 g, 57 mmol) in THF (300 ml) was added dropwise over a 90 minute period and the reaction mixture was stirred overnight at ambient temperature. THF was evaporated under reduced pressure and the remaining aqueous solution was lyophilized. The resultant crude product was dissolved in water (100 ml) and loaded onto a column of 1500 ml Bio-Rex 70 (ammonium hydroxide cycle, pH 9.7). The column was eluted stepwise with 0.005 N ammonium hydroxide (1.5 l), 0.015 N ammonium hydroxide (2 l), 0.03 N ammonium hydroxide (5 l), and finally with 0.05 N ammonium hydroxide (10 l). The fractions eluted with 0.05 N ammonium hydroxide were

analyzed by thin layer chromatography, and those containing the desired product were combined and lyophilized to give 6′-N-benzyloxycarbonylnebramine (9.7 g, 44% yield).

B. Preparation of 2′-N-(S-$\gamma$-Amino-$\alpha$-hydroxybutyryl)nebramine

6′-N-Benzyloxycarbonylnebramine (880 mg, 2 mmol) and nickel (II) acetate tetrahydrate (1.99 g, 8 mmol) were dissolved in water (20 ml) and this solution was diluted with dimethylformamide (100 ml). To the resultant mixture was added N-(S-$\gamma$-benzyloxycarbonylamino-$\alpha$-hydroxybutyryloxy)succinimide (1.05 g, 3 mmol). After stirring overnight at room temperature, the solvent was evaporated under reduced pressure, the residue was dissolved in methanol and the solvent was again evaporated to remove traces of dimethylformamide. The resultant residue was dissolved in a 30:10:1 mixture of dichloromethane:methanol:ammonium hydroxide and was loaded on a silica gel column (Grace 950, 90 g) pre-washed with the same solvent mixture. The desired product was eluted from the column using the 30:10:1 mixture of dichloromethane:methanol:ammonium hydroxide as eluant. The fractions containing the desired product were combined and the solvent was evaporated. The residue was dissolved in a 1:1 mixture of water:dioxane, filtered and lyophilized to give 6′-N-benzyloxycarbonyl-2′-N-(S-$\gamma$-benzyloxycarbonylamino-$\alpha$-hydroxybutyryl)nebramine (1.06 g, 79% yield).

6′-N-Benzyloxycarbonyl-2′-N-(S-$\gamma$-benzyloxycarbonylamino-$\alpha$-hydroxybutyryl)nebramine (975 mg) was dissolved in a mixture of dioxane (20 ml), water (20 ml) and glacial acetic acid (1 ml). 5% palladium on charcoal (270 mg) was added and the mixture was hydrogenated at $2.76 \times 10^5$ Pa (40 p.s.i overnight at ambient temperature. The palladium catalyst was removed by filtration and the filtrate was concentrated under reduced pressure and then loaded onto a column of 200 ml Bio-Rex 70 (ammonium hydroxide cycle, pH 8.6). After first eluting with a gradient of 0.005 N ammonium hydroxide (2 l) and 0.4 N ammonium hydroxide (3 l), the desired product was eluted with 0.5 N ammonium hydroxide. The fractions containing the desired product were combined and lyophilized to yield 2′-N-(S-$\gamma$-amino-$\alpha$-hydroxybutyryl)nebramine (397 mg, 70% yield): c.m.r. ($D_2O$—$H_2O$, pH $\geq$10) $\delta$ 32.4, 33.3, 36.2, 37.7, 42.0, 48.8, 50.4, 51.0, 66.4, 70.5, 73.6, 76.7, 78.4, 86.3, 97.3, 175.9; f.d.m.s. m/e 408 (P+1); thin layer chromatography, $R_f$=0.23 using a 3:1:2 volume ratio of methanol:methylene chloride: concentrated ammonium hydroxide.

Example 5

Preparation of 2′-N-Ethylapramycin

Apramycin (1.08 g, 2.0 mmol) and nickel(II) acetate tetrahydrate (1.99 g, 8.0 mmol) were dissolved in water (20 ml) and the solution was diluted with dimethylformamide (100 ml). The resultant opaque blue-green mixture was stirred for one hour at ambient temperature and then ethyl iodide (3.12 g, 20 mmol) was added. After stirring overnight at ambient temperature, solvent was evaporated under reduced pressure. The residue was suspended in a 30:10:1 mixture of methanol:dichloromethane:concentrated ammonium hydroxide (40 ml) and was loaded onto a silica gel column (Grace 950, 150 g) prewashed with the same solvent mixture. The column was eluted stepwise with eight different mixtures of methanol:dichloromethane:concentrated ammonium hydroxide [(a) 30:10:1 (400 ml), (b) 30:10:3 (430 ml), (c) 30:10:5 (900 ml), (d) 30:10:6 (460 ml), (e) 30:10:7 (1 l.) (f) 30:10:8 (400 ml), (g) 30:10:10 (400 ml), (h) 30:10:15 (400 ml)]. The fractions containing the desired product were combined, concentrated and lyophilized, yielding 785 mg of crude product. This material was dissolved in water, the solution's pH was adjusted to 8.8 and then the solution was loaded on a column of 300 ml Bio-Rex 70 (ammonium hydroxide cycle, pH 9.8, prewashed with 0.001 N ammonium hydroxide (1 l)). The column was eluted with a linear gradient of 0.01 N ammonium hydroxide (2 l.) and 0.10 N ammonium hydroxide (2 l). Fractions containing the desired product were combined and lyophilized, yielding 2′-N-ethylapramycin (150 mg, 13% yield): c.m.r. ($D_2O$—$H_2O$, pH $\geq$10) $\delta$ 14.8, 30.8, 33.1, 36.8, 41.7, 50.4, 51.4, 53.5, 56.1, 61.8, 62.4, 66.3, 68.0, 71.4, 72.2, 73.8, 74.3, 76.9, 78.5, 88.5, 95.6, 96.4, 100.2; f.d.m.s. m/e 567 (P+); thin layer chromatography; $R_f$=0.25 using a 6:2:3 volume ratio of methanol:methylene chloride: concentrated ammonium hydroxide.

Example 6

Preparation of 2′-N-(n-Butyl)apramycin

Method 1

Apramycin (54 mg, 0.1 mmol) was dissolved in water (1 ml). Nickel acetate tetrahydrate (100 mg, 0.4 mmol) was added and this solution was diluted with dimethylformamide (5 ml). The mixture was treated with 1-iodo-n-butane (0.115 ml, 1 mmol) and this reaction mixture was stirred at room temperature for 4 days. Thin layer chromatographic analysis (developed in 6:2:3 methanol:dichloromethane:ammonium hydroxide solution, visualized by chlorox spray and heating followed by starch-iodine spray reagent) of the reaction mixture showed 2′-N-(n-butyl)apramycin as the major product ($R_f$=0.35) along with unreacted apramycin ($R_f$=0.18). Bioautography of another thin layer chromatography plate against *B. subtilis* X12 showed a zone of inhibition due to 2′-N-(n-butyl)apramycin.

Method 2

The reaction of Method 1 was repeated using 1-bromo-(n-butane) (0.11 ml, 1 mmol) instead of 1-iodo-(n-butane). This reaction appeared to give a lower yield of the desired 2′-N-(n-butyl)apramycin than did the reaction of Method 1.

Example 7

Preparation of 2′-N-Benzylapramycin

The reaction of Example 6, Method 1, was followed, except that benzyl bromide (0.06 ml, 0.5 mmol) was used instead of 1-iodo-n-butane. The reaction mixture was stirred at room temperature for 19 h, at which time thin layer chromatographic analysis showed 2′-N-benzylapramycin ($R_5$=0.32) to be the major product. Bioautography of a second thin layer chromatography plate against *B. subtilis* X12 showed a zone of inhibition due to 2′-N-benzylapramycin.

Example 8

Preparation of 2′-N-Allylapramycin

Method 1

The reaction of Example 6, Method 1, was followed, except that allyl bromide (0.043 ml. 0.5 mmol) was used instead of 1-iodo-n-butane. The reaction mixture was stirred at room temperature for 22 h, at which time thin layer chromatographic analysis showed a substantial amount of 2′-N-allylapramycin ($R_f$=0.27) was present along with an unidentified material ($R_f$=0.38) and unreacted apramycin. Bioautography of a second thin layer chromatography plate against *B. subtilis* X12 showed a good zone of inhibition due to 2′-N-allylapramycin.

Method 2

The reaction of Method 1 above was repeated, except that allyl iodide (0.05 ml, 0.55 mmol) was used instead of allyl bromide. The reaction mixture was stirred at room temperature for 22 h, when thin layer chromatographic analysis of the reaction mixture gave the same results as in Method 1.

**Claims for the Contracting States BE CH DE FR GB IT LI LU NL SE:**

1. A 2′-N-monoacylated or 2′-N-monoalkylated derivative of a 4-O-substituted-2-deoxystreptamine aminoglycoside selected from the group consisting of;

apramycin,

α or β-($C_1$ to $C_6$ alkyl)aprosaminide,

α or β-($C_2$ to $C_6$ hydroxyalkyl)aprosaminide,

α or β-($C_2$ to $C_6$ aminoalkyl)aprosaminide,

α or β-($C_2$ to $C_6$ protected aminoalkyl)aprosaminide, α or β-($C_3$ to $C_6$ aminohydroxyalkyl)aprosaminide, α or β-($C_3$ to $C_6$ (protected amino)hydroxyalkyl)aprosaminide, or a pharmaceutically-acceptable acid addition salt thereof;

wherein the acyl or alkyl substituent group is selected from the group consisting of

γ-amino-α-hydroxybutyryl, γ-protected amino-α-hydroxybutyryl, β-amino-α-hydroxypropionyl, β-protected amino-α-hydroxypropionyl, glycyl, N-protected glycyl, 3-hydroxypyrrolidine-3-carbonyl, N-protected 3-hydroxypyrrolidine-3-carbonyl, 3-hydroxyazetidine-3-carbonyl, N-protected 3-hydroxyazetidine-3-carbonyl, 2-hydroxy-2-(azetidin-3-yl)acetyl, N-protected 2-hydroxy-2-(azetidin-3-yl)acetyl, 4-amino-2-hydroxybutyl, 3-amino-2-hydroxypropyl, 2-aminoethyl, 3-hydroxypyrrolidine-3-methylene, 3-hydroxyazetidine-3-methylene, 2-hydroxy-2-(azetidin-3-yl)ethyl, $C_2$ to $C_4$ alkyl, allyl, or alkylallyl having 3—5 carbon atoms, benzyl and substituted benzyl.

2. A 2′-N-acylated or 2′-N-alkylated derivative of a 4-O-substituted-2-deoxystreptomine aminoglycoside as claimed in claim 1 selected from:

2′-N-(S-γ-amino-α-hydroxybutyryl)apramycin,

2′-N-(RS-γ-amino-α-hydroxybutyryl)apramycin,

2′-N-glycylapramycin,

2′-N-ethylapramycin,

2′-N-(n-butyl)apramycin,

2′-N-benzylapramycin, and

2′-N-allylapramycin.

3. A 2′-N-acylated or 2′-N-alkylated derivative of a 4-O-substituted-2-deoxystreptamine aminoglycoside as claimed in claim 1 excluding those bearing protected amino or N-protected substituents.

4. A pharmaceutical formulation which comprises as an active ingredient a 2′-N-acylated or 2′-N-alkylated derivative of a 4-O-substituted-2-deoxystreptamine aminoglycoside as claimed in claim 3 associated with at least one carrier or diluent therefor.

5. A veterinary formulation which comprises as an active ingredient a 2′-N-acylated or 2′-N-alkylated derivated of a 4-O-substituted-2-deoxystreptamine aminoglycoside as claimed in claim 3 associated with at least one carrier or diluent therefor.

6. A surface disinfectant formulation which comprises as an active ingredient a 2′-N-acylated or 2′-N-alkylated derivative of a 4-O-substituted-2-deoxystreptamine aminoglycoside as claimed in claim 3 associated with at least one carrier or diluent therefor.

7. A process for preparing a 2′-N-monoacylated or 2′-N-monoalkylated derivative of a 4-O-substituted 2-deoxystreptamine aminoglycoside as defined in claim 1 or any one of the group lividamine, paromamine and the 6′-N-protected derivatives of nebramine, neamine, gentamine $C_1$, gentamine $C_{1a}$, gentamine $C_2$, gentamine $C_{2a}$, and gentamine $C_{2b}$, or a pharmaceutically-acceptable acid addition salt thereof which comprises the steps of:

(1) combining an aminoglycoside substrate selected from the group consisting of:

apramycin,

$\alpha$ or $\beta$-($C_1$ to $C_6$ alkyl)aprosaminide, $\alpha$ or $\beta$-($C_2$ to $C_6$ hydroxyalkyl)aprosaminide, $\alpha$ or $\beta$-($C_2$ to $C_6$ protected aminoalkyl)aprosaminide, $\alpha$ or $\beta$-($C_3$ to $C_6$ (protected amino)hydroxyalkyl)aprosaminide, lividamine, paromamine and the 6′-N-protected derivatives of nebramine, neamine, gentamine $C_1$, gentamine $C_{1a}$, gentamine $C_2$ gentamine $C_{2a}$, and gentamine $C_{2b}$;

with two to six molar equivalents of a nickel salt of the formula:

$$Ni(X)_2$$

in water;

(2) diluting the above aqueous mixture with between two and ten volumes of a polar organic solvent, and;

(3) adding between one and two molar equivalents of an acylating agent or between five and twenty molar equivalents of an alkylating agent to the above mixture of step (2) then carrying out the reaction at a temperature between 0°C. and 40°C. for a period between 0.5 and 24 hours for acylation or between 15 hours and 60 hours for alkylation, wherein:

X is $C_1$ to $C_{10}$ carboxylate;

the acylating agent used being a reactive derivative of an acyl moiety selected from the group consisting of $\gamma$-protected amino-$\alpha$-hydroxybutyryl, $\beta$-protected amino-$\alpha$-hydroxypropionyl, N-protected glycyl, N-protected 3-hydroxypyrrolidine-3-carbonyl, N-protected 3-hydroxy-azetidine-3-carbonyl and N-protected 2-hydroxy-2-(azetidin-3-yl)acetyl; and

the alkylating agents used being $C_2$ to $C_4$ alkyl bromide, $C_2$ to $C_4$ alkyl iodide, allyl or alkylallyl bromide having 3—5 carbon atoms, allyl or alkylallyl iodide having 3—5 carbon atoms, benzyl bromide, benzyl iodide, substituted benzyl bromide and substituted benzyl iodide; and

(4) optionally, deprotecting protected amino or N-protected substituents;

(5) optionally, reducing the carbonyl portion of an acyl substituent with a reducing agent;

(6) recovering as a free base or as a pharmaceutically-acceptable acid addition salt thereof.

8. A process as claimed in claim 6 wherein the product is selected from:

2′-N-(S-$\gamma$-amino-$\alpha$-hydroxybutyryl)apramycin,

2′-N-(RS-$\gamma$-amino-$\alpha$-hydroxybutyryl)apramycin,

2′-N-glycylapramycin,

2′-N-ethylapramycin,

2′-N-(n-butyl)apramycin,

2′-N-benzylapramycin,

2′-N-allylapramycin, and

2′-N-(S-$\gamma$-amino-$\alpha$-hydroxybutyryl)nebramine.

9. A 2′-N-acylated or a 2′-N-alkylated derivative of a 4-O-substituted 2-deoxystreptamine aminoglycoside as defined in claim 3 for use in treating warm-blooded animals having bacterial infections.

10. A 2′-N-acylated or a 2′-N-alkylated derivative of a 4-O-substituted 2-deoxystreptamine aminoglycoside as defined in claim 3 for use as a surface disinfectant.

11. A 2′-N-acylated or a 2′-N-alkylated derivative of a 4-O-substituted 2-deoxystreptamine aminoglycoside as defined in claim 3, for use as a pharmaceutical.

**Claims for the Contracting State AT:**

1. A process for preparing a 2′-N-monoacylated or a 2′-N-monoalkylated derivative of a 4-O-substituted-2-deoxystreptamine aminoglycoside selected from the group consisting of;

apramycin,

$\alpha$ or $\beta$-($C_1$ to $C_6$ alkyl)aprosaminide,

$\alpha$ or $\beta$-($C_2$ to $C_6$ hydroxyalkyl)aprosaminide,

$\alpha$ or $\beta$-($C_2$ to $C_6$ aminoalkyl)aprosaminide,

$\alpha$ or $\beta$-($C_2$ to $C_6$ protected aminoalkyl)aprosaminide, $\alpha$ or $\beta$-($C_3$ to $C_6$ aminohydroxyalkyl)aprosaminide, $\alpha$ or $\beta$-($C_3$ to $C_6$ (protected amino)hydroxyalkyl)aprosaminide, or a pharmaceutically-acceptable acid addition salt thereof;

wherein the acyl or alkyl substituent group is selected from the group consisting of

$\gamma$-amino-$\alpha$-hydroxybutyryl, $\gamma$-protected amino-$\alpha$-hydroxybutyryl, $\beta$-amino-$\alpha$-hydroxypropionyl, $\beta$-protected amino-$\alpha$-hydroxypropionyl, glycyl, N-protected glycyl, 3-hydroxypyrrolidine-3-carbonyl, N-protected 3-hydroxypyrrolidine-3-carbonyl, 3-hydroxyazetidine-3-carbonyl, N-protected 3-hydroxyazetidine-3-carbonyl, 2-hydroxy-2-(azetidin-3-yl)acetyl, N-protected 2-hydroxy-2-(azetidin-3-yl)acetyl, 4-amino-2-hydroxybutyl, 3-amino-2-hydroxypropyl, 2-aminoethyl, 3-hydroxypyrrolidine-3-methylene, 3-hydroxyazetidine-3-methylene, 2-hydroxy-2-(azetidin-3-yl)ethyl, $C_2$ to $C_4$ alkyl, allyl or alkylallyl having 3—5 carbon atoms, benzyl and substituted benzyl, or any one of the group lividamine, paromamine and the 6′-N-protected derivatives of nebramine, neamine, gentamine $C_1$, gentamine $C_{1a}$, gentamine $C_2$, gentamine $C_{2a}$, and gentamine $C_{2b}$, or a pharmaceutically-acceptable acid addition salt thereof which comprises the steps of:

(1) combining an aminoglycoside substrate selected from the group consisting of:
apramycin
$\alpha$ or $\beta$-($C_1$ to $C_6$ alkyl)aprosaminide, $\alpha$ or $\beta$-($C_2$ to $C_6$ hydroxyalkyl)aprosaminide, $\alpha$ or $\beta$-($C_2$ to $C_6$ protected aminoalkyl)aprosaminide, $\alpha$ or $\beta$-($C_3$ to $C_6$ (protected amino)hydroxyalkyl)aprosaminide, lividamine, paromamine and the 6′-N-protected derivatives of nebramine, neamine, gentamine $C_1$, gentamine $C_{1a}$, gentamine $C_2$ gentamine $C_{2a}$, and gentamine $C_{2b}$;

with two to six molar equivalents of a nickel salt of the formula:

$$Ni(X)_2$$

in water;

(2) diluting the above aqueous mixture with between two and ten volumes of a polar organic solvent, and;

(3) adding between one and two molar equivalents of an acylating agent or between five and twenty molar equivalents of an alkylating agent to the above mixture of step (2) then carrying out the reaction at a temperature between 0°C. and 40°C. for a period between 0.5 and 24 hours for acylation or between 15 hours and 60 hours for alkylation, wherein:

X is $C_1$ to $C_{10}$ carboxylate;

the acylating agent used being a reactive derivative of an acyl moiety selected from the group consisting of $\gamma$-protected amino-$\alpha$-hydroxybutyryl, $\beta$-protected amino-$\alpha$-hydroxypropionyl, N-protected glycyl, N-protected 3-hydroxypyrrolidine-3-carbonyl, N-protected 3-hydroxyazetidine-3-carbonyl and N-protected 2-hydroxy-2-(azetidin-3-yl)acetyl; and

the alkylating agents used being $C_2$ to $C_4$ alkyl bromide, $C_2$ to $C_4$ alkyl iodide, allyl or alkylallyl bromide having 3—5 carbon atoms, allyl or alkylallyl iodide having 3—5 carbon atoms, benzyl bromide, benzyl iodide, substituted benzyl bromide and substituted benzyl iodide; and

(4) optionally, deprotecting protected amino or N-protected substituents;

(5) optionally, reducing the carbonyl portion of an acyl substituent with a reducing agent;

(6) recovering as a free base or as a pharmaceutically-acceptable acid addition salt thereof.

2. A process of preparing 2′-N-(S-$\gamma$-amino-$\alpha$-hydroxybutyryl)apramycin as claimed in claim 1 which comprises reacting apramycin with nickel II acetate tetrahydrate in water, diluting the solution with dimethylformamide, treating with N-(S-$\gamma$-benzyloxycarbonylamino-$\alpha$-hydroxybutyryloxy)succinimide overnight to form 2′-N-(S-$\gamma$-benzyloxycarbonylamino-$\alpha$-hydroxybutyryl)apramycin, followed by removal of the protecting agent and recovering a free base.

3. A process of preparing a racemic mixture of 2′-N-(RS-$\gamma$-amino-$\alpha$-hydroxybutyryl)apramycin as claimed in claim 1 which comprises reacting apramycin with nickel II acetate tetrahydrate in water, diluting the solution with dimethylformamide, treating with a racemic mixture of N-(S-$\gamma$-benzyloxycarbonylamino-$\alpha$-hydroxybutyryloxy)succinimide overnight to form a racemic mixture of 2′-N-(2-$\gamma$-benzyloxycarbonylamino-$\alpha$-hydroxybutyryl)apramycin, followed by removal of the protecting agent and recovering a free base.

4. A process of preparing 2′-N-glycyl apramycin as claimed in claim 1 which comprises reacting apramycin with nickel II acetate tetrahydrate in water, diluting the solution with dimethylformamide, treating with N-(N-benzyloxy carbonylglycyloxy)succinimide overnight to form 2′-N-(N-benzyloxycarbonylglycyl)apramycin, followed by removal of the protecting agent and recovering as a free base.

5. A process of preparing 2′-N-(S-$\gamma$-amino-$\alpha$-hydroxy-butyryl)nebramine as claimed in claim 1 which comprises reacting 6′-N-benzyloxycarbonyl nebramine and nickel II acetate tetrahydrate in water, diluting with dimethylformamide, treating with N-(S-$\gamma$-benzyloxycarbonyl-amino-$\alpha$-hydroxybutyryloxy)succinimide overnight to give 6′-N-benzyloxycarbonyl-2′-N-S-$\gamma$-benzyloxycarbonylamino-$\alpha$-hydroxybutyryl)nebramine, followed by removal of the protecting agent and recovering as a free base.

6. A process of preparing 2′-N-ethylapramycin as claimed in claim 1 which comprises reacting apramycin and nickel II acetate tetrahydrate in water, diluting with dimethylformamide, and treating with ethyl iodide overnight and recovering the free base.

7. A process of preparing 2′-N-(n-butyl)apramycin as claimed in claim 1 which comprises reacting apramycin with nickel acetate tetrahydrate in water, diluting in dimethylformamide, treating with 1-iodo-n-butane for 4 days, and recovering as the free base.

8. A process of preparing 2′-N-benzylapramycin as claimed in claim 1 which comprises reacting apramycin with nickel acetate tetrahydrate in water, diluting in dimethylformamide, treating with benzyl bromide for 19 hours, and recovering as a free base.

9. A process for the preparation of 2′-N-allylapramycin as claimed in claim 1 which comprises reacting apramycin with nickel acetate tetrahydrate in water, diluting with dimethylformamide, treating with allyl bromide for 22 hours, and recovering as a free base.

**Patentansprüche für die Vertragsstaaten: BE CH DE FR GB IT LI LU NL SE**

1. 2′-N-Monoacyliertes oder 2′-N-monoalkyliertes Derivat eines 4-O-substituierten 2-Deoxystreptaminaminoglycosids ausgewählt aus der Gruppe
Apramycin
$\alpha$- oder $\beta$-($C_1$ bis $C_6$-Alkyl)aprosaminid,
$\alpha$- oder $\beta$-($C_2$ bis $C_6$-Hydroxyalkyl)aprosaminid,
$\alpha$- oder $\beta$-($C_2$ bis $C_6$-Aminoalkyl)aprosaminid,
$\alpha$- oder $\beta$-($C_2$ bis $C_6$-Geschütztes-Aminoalkyl)aprosaminid,
$\alpha$- oder $\beta$-($C_3$ bis $C_6$-Aminohydroxyalkyl)aprosaminid,
$\alpha$- oder $\beta$-($C_3$ bis $C_6$-(Geschütztes-Amino)hydroxyalkyl)aprosaminid,

oder einem pharmazeutisch annehmbaren Säureadditionssalz hiervon,

worin die Acyl- oder Alkylsubstituentengruppe ausgewählt ist aus der Gruppe $\gamma$-Amino-$\alpha$-hydroxybutyryl, $\gamma$-geschütztes Amino-$\alpha$-hydroxybutyryl, $\beta$-Amino-$\alpha$-hydroxypropionyl, $\beta$-geschütztes Amino-$\alpha$-hydroxypropionyl, Glycyl, N-geschütztes Glycyl, 3-Hydroxypyrrolidin-3-carbonyl, N-geschütztes 3-Hydroxypyrrolidin-3-carbonyl, 3-Hydroxyazetidin-3-carbonyl, N-geschütztes 3-Hydroxyazetidin-3-carbonyl, 2-Hydroxy-2-(azetidin-3-yl)acetyl, N-geschütztes 2-Hydroxy-2-(azetidin-3-yl)acetyl, 4-Amino-2-hydroxybutyl, 3-Amino-2-hydroxypropyl, 2-Aminoethyl, 3-Hydroxypyrrolidin-3-methylen, 3-Hydroxyazetidin-3-methylen, 2-Hydroxy-2-(azetidin-2-yl)ethyl, $C_2$ bis $C_4$-Alkyl, Allyl oder Alkylallyl mit 3 bis 5 Kohlenstoffatomen, Benzyl und substituiertes Benzyl.

2. 2′-N-Acyliertes oder 2′-N-alkyliertes Derivat eines 4-O-substituierten 2-Deoxystreptaminaminoglycosids nach Anspruch 1 ausgewählt aus:
2′-N-(S-$\gamma$-Amino-$\alpha$-hydroxybutyryl)apramycin,
2′-N-(RS-$\gamma$-Amino-$\alpha$-hydroxybutyryl)apramycin,
2′-N-Glycylapramycin,
2′-N-Ethylapramycin,
2′-N-(n-Butyl)apramycin,
2′-N-Benzylapramycin und
2′-N-Allylapramycin.

3. 2′-N-Acyliertes oder 2′-N-alkyliertes Derivat eines 4-O-substituierten 2-Deoxystreptaminaminoglycosids nach Anspruch 1, dadurch gekennzeichnet, daß die Verbindungen ausgeschlossen sind, die geschützte Aminosubstituenten oder N-geschützte Substituenten enthalten.

4. Pharmazeutische Formulierung, dadurch gekennzeichnet, daß sie ein 2′-N-acyliertes oder 2′-N-alkyliertes Derivat eines 4-O-substituierten 2-Deoxystreptaminaminoglycosids nach Anspruch 3 als Wirkstoff in Verbindung mit wenigstens einem Träger oder Verdünnungsmittel hierfür enthält.

5. Veterinärmedizinische Formulierung, dadurch gekennzeichnet, daß sie ein 2′-N-acyliertes oder 2′-N-alkyliertes Derivat eines 4-O-substituierten 2-Deoxystreptaminaminoglycosids nach Anspruch 3 als Wirkstoff in Verbindung mit wenigstens einem Träger oder Verdünnungsmittel hierfür enthält.

6. Oberflächendesinfizierende Formulierung, dadurch gekennzeichnet, daß sie ein 2′-N-acyliertes oder 2′-N-alkyliertes Derivat eines 4-O-substituierten 2-Deoxystreptaminaminoglycosids nach Anspruch 3 als Wirkstoff in Verbindung mit wenigstens einem Träger oder Verdünnungsmittel hierfür enthält.

7. Verfahren zur Herstellung eines 2′-N-monoacylierten oder 2′-N-monoalkylierten Derivats eines 4-O-substituierten 2-Deoxystreptaminaminoglycosids nach Anspruch 1 oder irgendeine Verbindung aus der Gruppe Lividamin, Paromamin und der 6′-N-geschützten Derivate von Nebramin, Neamin, Gentamin-$C_1$, Gentamin-$C_{1a}$, Gentamin-$C_2$, Gentamin-$C_{2a}$ und Gentamin $C_{2b}$ oder eines pharmazeutisch annehmbaren Säureadditionssalzes hiervon, dadurch gekennzeichnet, daß man

(1) ein Aminoglycosidsubstrat ausgewählt aus der Gruppe
Apramycin
$\alpha$- oder $\beta$-($C_1$ bis $C_6$-Alkyl)aprosaminid, $\alpha$- oder $\beta$-($C_2$ bis $C_6$-Hydroxyalkyl)aprosaminid, $\alpha$- oder $\beta$-($C_2$ bis $C_6$-Geschütztes-aminoalkyl)aprosaminid, $\alpha$- oder $\beta$-($C_3$ bis $C_6$-(Geschütztes-amino)hydroxyalkyl)aprosaminid, Lividamin, Paromamin und den 6′-N-geschützten Derivaten von Nebramin, Neamin, Gentamin-$C_1$, Gentamin-$C_{1a}$, Gentamin-$C_2$, Gentamin-$C_{2a}$ und Gentamin-$C_{2b}$,

mit zwei bis sechs Moläquivalent eines Nickelsalzes der Formel

$$Ni(X)_2$$

in Wasser vereinigt,

(2) das obige wässrige Gemisch mit zwei bis zehn Volumina eines polaren organischen Lösungsmittels verdünnt und

(3) das obige Gemisch der Stufe (2) mit eins bis zwei Moläquivalenten eines Acylierungsmittels oder mit fünf bis zwanzig Moläquivalenten eines Alkylierungsmittels versetzt und dann die Umsetzung bei einer Temperatur zwischen 0°C und 40°C während einer Zeitdauer von 0,5 bis 24 Stunden zur Acylierung oder von 15 bis 60 Stunden zur Alkylierung durchführt,

worin X für ein $C_1$ bis $C_{10}$-Carboxylat steht,

wobei das verwendete Acylierungsmittel ein reaktionsfähiges Derivat eines Acylrests ausgewählt aus der Gruppe von $\gamma$-geschütztem Amino-$\alpha$-hydroxybutyryl, $\beta$-geschütztem Amino-$\alpha$-hydroxypropionyl, N-geschütztem Glycyl, N-geschütztem 3-Hydroxypyrrolidin-3-carbonyl, N-geschütztem 3-Hydroxyazetidin-3-carbonyl und N-geschütztem 2-Hydroxy-2-(azetidin-3-yl)acetyl ist und

die verwendeten Alkylierungsmittel $C_2$ bis $C_4$-Alkylbromid, $C_2$ bis $C_4$-Alkyliodid, Allylbromid oder Alkylallylbromid mit 3 bis 5 Kohlenstoffatomen, Allyliodid oder Alkylallyliodid mit 3 bis 5 Kohlenstoffatomen, Benzylbromid, Benzyliodid, substituiertes Benzylbromid und substituiertes Benzyliodid sind und

(4) geschützte Aminosubstituenten oder N-geschützte Substituenten gegebenenfalls von Schutzgruppen befreit,

(5) den Carbonylrest eines Acylsubstituenten gegebenenfalls mit einem Reduktionsmittel reduziert und

(6) die gewünschte Verbindung als freie Base oder als pharmazeutisch annehmbares Säureadditionssalz gewinnt.

8. Verfahren nach Anspruch 6, dadurch gekennzeichnet, daß das Produkt ausgewählt ist aus
2′-N-(S-$\gamma$-Amino-$\alpha$-hydroxybutyryl)apramycin,
2′-N-(RS-$\gamma$-Amino-$\alpha$-hydroxybutyryl)apramycin,
2′-N-Glycylapramycin,
2′-N-Ethylapramycin,
2′-N-(n-Butyl)apramycin,
2′-N-Benzylapramycin,
2′-N-Allylapramycin und
2′-N-(S-$\gamma$-Amino-$\alpha$-hydroxybutyryl)nebramin.

9. Verwendung eines 2′-N-acylierten oder eines 2′-N-alkylierten Derivats eines 4-O-substituierten 2-Deoxystreptaminaminoglycosids nach Anspruch 1 zur Behandlung von Warmblütern mit Bakterieninfektionen.

10. Verwendung eines 2′-N-acylierten oder eines 2′-N-alkylierten Derivats eines 4-O-substituierten 2-Deoxystreptaminaminoglycosids nach Anspruch 1 als oberflächendesinfizierendes Mittel.

11. Verwendung eines 2′-N-acylierten oder eines 2′-N-alkylierten Derivats eines 4-O-substituierten 2-Deoxystreptaminaminoglycosids nach Anspruch 3 als Pharmazeutikum.

**Patentansprüche für den Vertragsstaat: AT**

1. Verfahren zur Herstellung eines 2′-N-monoacylierten oder eines 2′-N-monoalkylierten Derivats eines 4-O-substituierten 2-Deoxystreptaminaminoglycosids ausgewählt aus der Gruppe
Apramycin
$\alpha$- oder $\beta$-($C_1$ bis $C_6$-Alkyl)aprosaminid,
$\alpha$- oder $\beta$-($C_2$ bis $C_6$-Hydroxyalkyl)aprosaminid,
$\alpha$- oder $\beta$-($C_2$ bis $C_6$-Aminoalkyl)aprosaminid,
$\alpha$- oder $\beta$-($C_2$ bis $C_6$-Geschütztes-Aminoalkyl)aprosaminid,
$\alpha$- oder $\beta$-($C_3$ bis $C_6$-Aminohydroxyalkyl)aprosaminid,
$\alpha$- oder $\beta$-($C_3$ bis $C_6$-(Geschütztes-Amino)hydroxyalkyl)aprosaminid,

oder einem pharmazeutisch annehmbaren Säureadditionssalz hiervon,

worin die Acyl- oder Alkylsubstituentengruppe ausgewählt ist aus der Gruppe $\gamma$-Amino-$\alpha$-hydroxybutyryl, $\gamma$-geschütztes Amino-$\alpha$-hydroxybutyryl, $\beta$-Amino-$\alpha$-hydroxypropionyl, $\beta$-geschütztes Amino-$\alpha$-hydroxypropionyl, Glycyl, N-geschütztes Glycyl, 3-Hydroxypyrrolidin-3-carbonyl, N-geschütztes 3-Hydroxypyrrolidin-3-carbonyl, 3-Hydroxyazetidin-3-carbonyl, N-geschütztes 3-Hydroxyazetidin-3-carbonyl, 2-Hydroxy-2-(azetidin-3-yl)acetyl, N-geschütztes 2-Hydroxy-2-(azetidin-3-yl)acetyl, 4-Amino-2-hydroxybutyl, 3-Amino-2-hydroxypropyl, 2-Aminoethyl, 3-Hydroxypyrrolidin-3-methylen, 3-Hydroxyazetidin-3-methylen, 2-Hydroxy-2-(azetidin-2-yl)ethyl, $C_2$ bis $C_4$-Alkyl, Allyl oder Alkylallyl mit 3 bis 5 Kohlenstoffatomen, Benzyl und substituiertes Benzyl oder irgendeine Verbindung aus der Gruppe Lividamin Paromamin und der 6′-N-geschützten Derivate von Nebramin, Neamin, Gentamin-$C_1$, Gentamin-$C_{1a}$, Gentamin-$C_2$, Gentamin-$C_{2a}$ und Gentamin $C_{2b}$ oder eines pharmazeutisch annehmbaren Säureadditionssalzes hiervon, dadurch gekennzeichnet, daß man

(1) eine Aminoglycosidsubstrat ausgewählt aus der Gruppe
Apramycin
$\alpha$- oder $\beta$-($C_1$ bis $C_6$-Alkyl)aprosaminid, $\alpha$- oder $\beta$-($C_2$ bis $C_6$-Hydroxyalkyl)aprosaminid, $\alpha$- oder $\beta$-

($C_2$ bis $C_6$-Geschütztes-aminoalkyl)aprosaminid, $\alpha$- oder $\beta$-($C_3$ bis $C_6$-(Geschütztes-amino)hydroxyalkyl)aprosaminid, Lividamin, Paromamin und den 6'-N-geschützten Derivaten von Nebramin, Neamin, Gentamin-$C_1$, Gentamin-$C_{1a}$, Gentamin-$C_2$, Gentamin-$C_{2a}$ und Gentamin-$C_{2b}$,

mit zwei bis sechs Moläquivalent eines Nickelsalzes der Formel

$$Ni(X)_2$$

in Wasser vereinigt,

(2) das obige wässrige Gemisch mit zwei bis zehn Volumina eines polaren organischen Lösungsmittels verdünnt und

(3) das obige Gemisch der Stufe (2) mit eins bis zwei Moläquivalenten eines Acylierungsmittels oder mit fünf bis zwanzig Moläquivalenten eines Alkylierungsmittels versetzt und dann die Umsetzung bei einer Temperatur zwischen 0°C und 40°C während einer Zeitdauer von 0,5 bis 24 Stunden zur Acylierung oder von 15 bis 60 Stunden zur Alkylierung durchführt,

worin X für ein $C_1$ bis $C_{10}$-Carboxylat steht,

wobei das verwendete Acylierungsmittel ein reaktionsfähiges Derivat eines Acylrests ausgewählt aus der Gruppe von $\gamma$-geschütztem Amino-$\alpha$-hydroxybutyryl, $\beta$-geschütztem Amino-$\alpha$-hydroxypropionyl, N-geschütztem Glycyl, N-geschütztem 3-Hydroxypyrrolidin-3-carbonyl, N-geschütztem 3-Hydroxyazetidin-3-carbonyl und N-geschütztem 2-Hydroxy-2-(azetidin-3-yl)acetyl ist und

die verwendeten Alkylierungsmittel $C_2$ bis $C_4$-Alkylbromid, $C_2$ bis $C_4$-Alkyliodid, Allylbromid oder Alkylallylbromid mit 3 bis 5 Kohlenstoffatomen, Allyliodid oder Alkylallyliodid mit 3 bis 5 Kohlenstoffatomen, Benzylbromid, Benzyliodid, substituiertes Benzylbromid und substituiertes Benzyliodid sind und

(4) geschützte Aminosubstituenten oder N-geschützte Substituenten gegebenenfalls von Schutzgruppen befreit,

(5) den Carbonylrest eines Acylsubstituenten gegebenenfalls mit einem Reduktionsmittel reduziert und

(6) die gewünschte Verbindung als freie Base oder als pharmazeutisch annehmbares Säureadditionssalz gewinnt.

2. Verfahren zur Herstellung von 2'N-(S-$\gamma$-Amino-$\alpha$-hydroxybutyryl)apramycin nach Anspruch 1, dadurch gekennzeichnet, daß man Apramycin mit Nickel (II)-acetattetrahydrat in Wasser umsetzt, die Lösung mit Dimethylformamid verdünnt, über Nacht mit N-(S-$\gamma$-Benzyloxycarbonylamino-$\alpha$-hydroxybutyryloxy)succinimid behandelt und so 2'-N-(S-$\gamma$-Benzyloxycarbonylamino-$\alpha$-hydroxybutyryl)apramycin bildet, dann die Schutzgruppen entfernt und die freie Base gewinnt.

3. Verfahren zur Herstellung eines racemischen Gemisches aus 2'N-(RS-$\gamma$-Amino-$\alpha$-hydroxybutyryl)apramycin nach Anspruch 1, dadurch gekennzeichnet, daß man Apramycin und Nickel(II)-acetattetrahydrat in Wasser umsetzt, die Lösung mit Dimethylformamid verdünnt, über Nacht mit einem racemischen Gemisch von N-(S-$\gamma$-Benzyloxycarbonylamino-$\alpha$-hydroxybutyryloxy)succinimid behandelt und so ein racemisches Gemisch von 2'-N-(S-$\gamma$-Benzyloxycarbonylamino-$\alpha$-hydroxybutyryl)apramycin bildet, dann die Schutzgruppen entfernt und die freie Base gewinnt.

4. Verfahren zur Herstellung von 2'-N-Glycylapramycin nach Anspruch 1, dadurch gekennzeichnet, daß man Apramycin mit Nickel(II)-acetattetrahydrat in Wasser umsetzt, die Lösung mit Dimethylformamid verdünnt, über Nacht mit N-(N-Benzyloxycarbonylglycyloxy)succinimid behandelt und so 2'-N-(N-Benzyloxycarbonylglycyl)apramycin bildet, dann die Schutzgruppen entfernt und die freie Base gewinnt.

5. Verfahren zur Herstellung von 2'-N-(S-$\gamma$-Amino-$\alpha$-hydroxybutyryl)nebramin nach Anspruch 1, dadurch gekennzeichnet, daß man 6'-N-Benzyloxycarbonylnebramin mit Nickel(II)-acetattetrahydrat in Wasser umsetzt, mit Dimethylformamid verdünnt, über Nacht mit N-(S-$\gamma$-Benzyloxycarbonylamino-$\alpha$-hydroxybutyryloxy)succinimid behandelt und so 6'-N-Benzyloxycarbonyl-2'-N-S-$\gamma$-benzyloxycarbonylamino-$\alpha$-hydroxybutyryl)nebramin bildet, dann die Schutzgruppen entfernt und die freie Base gewinnt.

6. Verfahren zur Herstellung von 2'-N-Ethylapramycin nach Anspruch 1, dadurch gekennzeichnet, daß man Apramycin mit Nickel(II)-acetattetrahydrat in Wasser umsetzt, mit Dimethylformamid verdünnt, über Nacht mit Ethyliodid behandelt und die freie Base gewinnt.

7. Verfahren zur Herstellung von 2'-N-(n-Butyl)apramycin nach Anspruch 1, dadurch gekennzeichnet, daß man Apramycin mit Nickel(II)-acetattetrahydrat in Wasser umsetzt, mit Dimethylformamid verdünnt, 4 Tage mit 1-Iod-n-butan behandelt und die freie Base gewinnt.

8. Verfahren zur Herstellung von 2'-N-Benzylapramycin nach Anspruch 1, dadurch gekennzeichnet, daß man Apramycin mit Nickel(II)-acetattetrahydrat in Wasser umsetzt, mit Dimethylformamid verdünnt, 19 Stunden mit Benzylbromid behandelt und die freie Base gewinnt.

9. Verfahren zur Herstellung von 2'-N-Allylapramycin nach Anspruch 1, dadurch gekennzeichnet, daß man Apramycin mit Nickel(II)-acetattetrahydrat in Wasser umsetzt, mit Dimethylformamid verdünnt, 22 Stunden mit Allylbromid behandelt und die freie Base gewinnt.

**Revendications pour les Etats Contractants: BE CH DE FR GB IT LI LU NL SE**

1. Dérivé 2′-N-mono-acylé ou 2′-N-monoalkylé d'un 2-désoxystreptamine-aminoglycoside 4-O-substitué choisi parmi le groupe comprenant:

1′apramycine,

1′α- ou β-(alkyl en $C_1$—$C_6$)aprosaminide,

1′α- ou β-(hydroxyalkyl en $C_2$—$C_6$)aprosaminide,

1′α- ou β-(aminoalkyl en $C_2$—$C_6$)aprosaminide,

1′α- ou β-((amino protégé)alkyl en $C_2$—$C_6$)aprosaminide,

1′α- ou β-(aminohydroxyalkyl en $C_3$—$C_6$)aprosaminide,

1′α- ou β-((amino protégé)hydroxyalkyl en $C_3$—$C_6$)aprosaminide ou un de leurs sels d'addition d'acide pharmaceutiquement acceptables;

caractérisé en ce que le groupe substituant acyle ou alkyle est choisi parmi le groupe comprenant: les groupes γ-amino-α-hydroxybutyryle, amino γ-protégé-α-hydroxybutyryle, β-amino-α-hydroxypropionyle, amino β-protégé-α-hydroxypropionyle, glycyle, glycyle N-protégé, 3-hydroxypyrrolidine-3-carbonyle, 3-hydroxypyrrolidine-3-carbonyle N-protégé, 3-hydroxyazétidine-3-carbonyle, 3-hydroxyazétidine-3-carbonyle N-protégé, 2-hydroxy-2-(azétin-3-yl)acétyle, 2-hydroxy-2-(azétidin-3-yl)acétyle N-protégé, 4-amino-2-hydroxybutyle, 3-amino-2-hydroxypropyle, 2-aminoéthyle, 3-hydroxypyrrolidine-3-méthylène, 3-hydroxyazétidine-3-méthylène, 2-hydroxy-2-(azétidin-3-yl)éthyle, alkyle en $C_2$—$C_4$, allyle ou alkyl-allyle contenant 3 à 5 atomes de carbone, benzyle et benzyle substitué.

2. Dérivé 2′-N-acylé ou 2′-N-alkylé d'un 2-déoxystreptamine-aminoglycoside 4-O-substitué suivant la revendication 1, choisi parmi:

la 2′-N-(S-γ-amino-α-hydroxybutyryl)apramycine,

la 2′-N-(RS-γ-amino-α-hydroxybutyryl)apramycine,

la 2′-N-glycylapramycine,

la 2′-N-éthylapramycine,

la 2′-N-(n-butyl)apramycine,

la 2′-N-benzylapramycine, et

la 2′-N-allylapramycine.

3. Dérivé 2′-N-acylé ou 2′-N-alkylé d'un 2-désoxystreptamine-aminoglycoside 4-O-substitué suivant la revendication 1, à l'exclusion de ceux comportant des substituants amino-protégés ou N-protégés.

4. Formulation pharmaceutique comprenant, comme ingrédient actif, un dérivé 2′-N-acylé ou 2′-N-alkylé d'un 2-désoxystreptamine-aminoglycoside 4-O-substitué suivant la revendication 3, en association avec au moins un support ou un diluant pour ce dérivé.

5. Formulation vétérinaire comprenant, comme ingrédient actif, un dérivé 2′-N-acylé ou 2′-N-alkylé d'un 2-désoxystreptamine-aminoglycoside 4-O-substitué suivant la revendication 3, en association avec au moins un support ou un diluant pour ce dérivé.

6. Formulation désinfectante superficielle comprenant, comme ingrédient actif, un dérivé 2′-N-acylé ou 2′-N-alkylé d'un 2-désoxystreptamine-aminoglycoside 4-O-substitué suivant la revendication 3, en association avec au moins un support ou un diluant pour ce dérivé.

7. Procédé de préparation d'un dérivé 2′-N-mono-acylé ou 2′-N-mono-alkylé d'un 2-désoxystreptamine-aminoglycoside 4-O-substitué suivant la revendication 1 ou de l'un ou l'autre des membres du groupe comprenant la lividamine, la paromamine et les dérivés 6′-N-protégés de la nébramine, de la néamine, de la gentamine $C_1$, de la gentamine $C_{1a}$, de la gentamine $C_2$, de la gentamine $C_{2a}$ et de la gentamine $C_{2b}$, ou d'un de leurs sels d'addition d'acide pharmaceutiquement acceptables, caractérisé en ce qu'il comprend les étapes qui consistent à:

(1) combiner un substrat d'aminoglycoside choisi parmi le groupe comprenant:

l'apramycine,

l'α- ou β-(alkyl en $C_1$—$C_6$)aprosaminide, l'α- ou β-(hydroxyalkyl en $C_2$—$C_6$)aprosaminide, l'α- ou β((amino protégé)alkyl en $C_2$—$C_6$)aprosaminide, l'α ou β-((amino protégé)hydroxyalkyl en $C_3$—$C_6$)aprosaminide, la lividamine, la paromamine et les dérivés 6′-N-protégés de la nébramine, de la néamine, de la gentamine $C_1$, de la gentamine $C_{1a}$, de la gentamine $C_2$, de la gentamine $C_{2a}$ et de la gentamine $C_{2b}$;

avec 2 à 6 équivalents molaires d'un sel de nickel de formule:

$$Ni(X)_2$$

dans l'eau;

(2) diluer le mélange aqueux ci-dessus avec entre 2 et 10 volumes d'un solvant organique polaire, et

(3) ajouter entre 1 et 2 équivalents molaires d'un agent d'acylation ou entre 5 et 20 équivalents

molaires d'un agent d'alkylation au mélange ci-dessus de l'étape (2), puis effectuer la réaction à une température comprise entre 0°C et 40°C pendant une période comprise entre 0,5 et 24 heures pour l'acylation ou entre 15 heures et 60 heures pour l'alkylation,

formule dans laquelle X représente un groupe carboxylate en $C_1$—$C_{10}$;

l'agent d'acylation utilisé étant un dérivé réactif d'une fraction acyle choisie parmi le groupe comprenant les groupes amino $\gamma$-protégé-$\alpha$-hydroxybutyryle, amino $\beta$-protégé-$\alpha$-hydroxypropionyle, glycyle N-protégé, 3-hydroxypyrrolidine-3-carbonyle N-protégé, 3-hydroxyazétidine-3-carbonyle N-protégé et 2-hydroxy-2-(azétidin-3-yl)acétyle N-protégé; et

les agents d'alkylation utilisés étant un bromure d'alkyle en $C_2$—$C_4$, un iodure d'alkyle en $C_2$—$C_4$, un bromure d'allyle ou d'alkyl-allyle contenant 3 à 5 atomes de carbone, un iodure d'allyle ou d'alkyl-allyle contenant 3 à 5 atomes de carbone, le bromure de benzyle, l'iodure de benzyle, le bromure de benzyle substitué et l'iodure de benzyle substitué; et

(4) éventuellement déprotéger les substituants amino-protégés ou N-protégés;

(5) éventuellement réduire la fraction carbonyle d'un substituant acyle avec un agent réducteur;

(6) récupérer sous forme d'une base libre ou sous forme d'un sel d'addition d'acide pharmaceutiquement acceptable.

8. Procédé suivant la revendication 6, caractérisé en ce que le produit est choisi parmi:
la 2'-N-(S-$\gamma$-amino-$\alpha$-hydroxybutyryl)apramycine,
la 2'-N-(RS-$\gamma$-amino-$\alpha$-hydroxybutyryl)apramycine,
la 2'-N-glycylapramycine,
la 2'-N-éthylapramycine,
la 2'-N-(n-butyl)apramycine,
la 2'-N-benzylapramycine,
la 2'-N-allylapramycine, et
la 2'-N-(S-$\gamma$-amino-$\alpha$-hydroxybutyryl)nébramine.

9. Dérivé 2'-N-acylé ou 2'-N-alkylé d'un 2-désoxystreptamine-aminoglycoside 4-O-substitué suivant la revendication 3 en vue de l'utiliser pour le traitement des animaux à sang chaud présentant des infections bactériennes.

10. Dérivé 2'-N-acylé ou 2'-N-alkylé d'un 2-désoxystreptamine-aminoglycoside 4-O-substitué suivant la revendication 3 en vue de l'utiliser comme désinfectant superficiel.

11. Dérivé 2'-N-acylé ou 2'-N-alkylé d'un 2-désoxystreptamine-aminoglycoside 4-O-substitué suivant la revendication 3, en vue de l'utiliser comme produit pharmaceutique.

## Revendications pour l'Etat Contractant: AT

1. Procédé de préparation d'un dérivé 2'-N-mono-acylé ou 2'-N-mono-alkylé d'un 2-désoxystreptamine-aminoglycoside 4-O-substitué choisi parmi le groupe comprenant:
l'apramycine,
l'$\alpha$- ou $\beta$-(alkyl en $C_1$—$C_6$)aprosaminide,
l'$\alpha$- ou $\beta$-(hydroxyalkyl en $C_2$—$C_6$)aprosaminide,
l'$\alpha$- ou $\beta$-(aminoalkyl en $C_2$—$C_6$)aprosaminide,
l'$\alpha$- ou $\beta$-((amino protégé)alkyl en $C_2$—$C_6$)aprosaminide, l'$\alpha$- ou $\beta$-(aminohydroxyalkyl en $C_3$—$C_6$)aprosaminide, l'$\alpha$- ou $\beta$-((amino protégé)hydroxyalkyl en $C_3$—$C_6$)aprosaminide ou un de leurs sels d'addition d'acide pharmaceutiquement acceptables;

où le groupe substituant acyle ou alkyle est choisi parmi le groupe comprenant:

les groupes $\gamma$-amino-$\alpha$-hydroxybutyryle, amino $\gamma$-protégé-$\alpha$-hydroxybutyryle, $\beta$-amino-$\alpha$-hydroxypropionyle, amino $\beta$-protégé-$\alpha$-hydroxypropionyle, glycyle, glycyle N-protégé, 3-hydroxypyrrolidine-3-carbonyle, 3-hydroxypyrrolidine-3-carbonyle N-protégé, 3-hydroxyazétidine-3-carbonyle, 3-hydroxyazétidine-3-carbonyle N-protégé, 2-hydroxy-2-(azétidin-3-yl)acétyle, 2-hydroxy-2-(azétidin-3-yl)acétyle N-protégé, 4-amino-2-hydroxybutyle, 3-amino-2-hydroxypropyle, 2-aminoéthyle, 3-hydroxypyrrolidine-3-méthylène, 3-hydroxyazétidine-3-méthylène, 2-hydroxy-2-(azétidin-3-yl)éthyle, alkyle en $C_2$—$C_4$, allyle ou alkyl-allyle contenant 3 à 5 atomes de carbone, benzyle et benzyle substitué, ou l'un ou l'autre des membres du groupe comprenant la lividamine, la paromamine et les dérivés 6'-N-protégés de la nébramine, de la néamine, de la gentamine $C_1$, de la gentamine $C_{1a}$, de la gentamine $C_2$, de la gentamine $C_{2a}$ et de la gentamine $C_{2b}$ ou un de leurs sels d'addition d'acide pharmaceutiquement acceptables, caractérisé en ce qu'il comprend les étapes qui consistent à:

(1) combiner un substrat d'aminoglycoside choisi parmi le groupe comprenant:
l'apramycine,
un $\alpha$- ou $\beta$-(alkyl en $C_1$—$C_6$)aprosaminide, un $\alpha$- ou $\beta$-(hydroxyalkyl en $C_2$—$C_6$)aprosaminide, un $\alpha$- ou $\beta$-((amino protégé)alkyl en $C_2$—$C_6$)aprosaminide, un $\alpha$- ou $\beta$-((amino protégé)hydroxyalkyl en $C_3$—$C_6$)aprosaminide, la lividamine, la paromamire et les dérivés 6'-N-protégés de la nébramine, de la néamine, de la gentamine $C_1$, de la gentamine $C_{1a}$, de la gentamine $C_2$, de la gentamine $C_{2a}$ et de la gentamine $C_{2b}$;

avec 2 à 6 équivalents molaires d'un sel de nickel de formule:

$$Ni(X)_2$$

dans l'eau;

(2) diluer le mélange aqueux ci-dessus avec entre 2 et 10 volumes d'un solvant organique polaire, et

(3) ajouter entre 1 et 2 équivalents molaires d'un agent d'acylation ou entre 5 et 20 équivalents molaires d'un agent d'alkylation au mélange ci-dessus de l'étape (2), puis effectuer la réaction à une température comprise entre 0°C et 40°C pendant une période comprise entre 0,5 et 24 heures pour l'acylation ou entre 15 heures et 60 heures pour l'alkylation,

X étant un groupe carboxylate en $C_1$—$C_{10}$;

l'agent d'acylation utilisé étant un dérivé réactif d'une fraction acyle choisie parmi le groupe comprenant les groupes amino $\gamma$-protégé-$\alpha$-hydroxybutyryle, amino $\beta$-protégé-$\alpha$-hydroxypropionyle, glycyle N-protégé, 3-hydroxypyrrolidine-3-carbonyle N-protégé, 3-hydroxy-azétidine-3-carbonyle N-protégé et 2-hydroxy-2-(azétidin-3-yl)acétyle N-protégé; et

les agents d'alkylation utilisés étant un bromure d'alkyle en $C_2$—$C_4$, un iodure d'alkyle en $C_2$—$C_4$, un bromure d'allyle ou d'alkyl-allyle contenant 3 à 5 atomes de carbone, un iodure d'allyle ou d'alkyl-allyle contenant 3 à 5 atomes de carbone, le bromure de benzyle, l'iodure de benzyle, le bromure de benzyle substitué et l'iodure de benzyle substitué; et

(4) éventuellement déprotéger les substituants amino-protégés ou N-protégés;

(5) éventuellement réduire la fraction carbonyle d'un substituant acyle avec un agent réducteur;

(6) récupérer sous forme d'une base libre ou sous forme d'un sel d'addition d'acide pharmaceutiquement acceptable.

2. Procédé de préparation de la 2'-N-(S-$\gamma$-amino-$\alpha$-hydroxybutyryl)apramycine suivant la revendication 1, caractérisé en ce qu'il consiste à faire réagir l'apramycine avec l'acétate de nickel(II) à 4 molécules d'eau dans l'eau, diluer la solution avec le diméthylformamide, effectuer un traitement avec le N-(S-$\gamma$-benzyloxycarbonylamino-$\alpha$-hydroxybutyryloxy)succinimide pendant une nuit pour former la 2'-N-(S-$\gamma$-benzyloxycarbonylamino-$\alpha$-hydroxybutyryl)apramycine, puis éliminer l'agent protecteur et récupérer une base libre.

3. Procédé de préparation d'un mélange racémique de la 2'-N-(RS-$\gamma$-amino-$\alpha$-hydroxybutyryl)apramycine suivant la revendication 1, caractérisé en ce qu'il consiste à faire réagir l'apramycine avec l'acétate de nickel(II) à 4 molécules d'eau dans l'eau, diluer la solution avec le diméthylformamide, effectuer un traitement avec un mélange racémique de N-(S-$\gamma$-benzyloxycarbonylamino-$\alpha$-hydroxybutyryloxy)succinimide pendant une nuit pour former un mélange racémique de 2'-N-(S-$\gamma$-benzyloxycarbonylamino-$\alpha$-hydroxybutyryl)apramycine, puis éliminer l'agent protecteur et récupérer une base libre.

4. Procédé de préparation de la 2'-N-glycylapramycine suivant la revendication 1, caractérisé en ce qu'il consiste à faire réagir l'apramycine avec l'acétate de nickel(II) à 4 molécules d'eau dans l'eau, diluer la solution avec le diméthylformamide, effectuer un traitement avec le N-(N-benzyloxy-carbonylglycyloxy)succinimide pendant une nuit pour former la 2'-N-(N-benzyloxycarbonylglycyl)apramycine, puis éliminer l'agent protecteur et récupérer une base libre.

5. Procédé de préparation de la 2'-N-(S-$\gamma$-amino-$\alpha$-hydroxybutyryl)nébramine suivant la revendication 1, caractérisé en ce qu'il consiste à faire réagir la 6'-N-benzyloxycarbonyl-nébramine et l'acétate de nickel(II) à 4 molécules d'eau dans l'eau, diluer avec le diméthylformamide, effectuer un traitement avec le N-(S-$\gamma$-benzyloxycarbonylamino-$\alpha$-hydroxybutyryloxy)succinimide pendant une nuit pour obtenir la 6'-N-benzyloxycarbonyl-2'-N-(S-$\gamma$-benzyloxycarbonylamino-$\alpha$-hydroxybutyryl)nébramine, puis éliminer l'agent protecteur et récupérer une base libre.

6. Procédé de préparation de la 2'-N-éthylapramycine suivant la revendication 1, caractérisé en ce qu'il consiste à faire réagir l'apramycine et l'acétate de nickel(II) à 4 molécules d'eau dans l'eau, diluer avec le diméthylformamide, effectuer un traitement avec l'iodure d'éthyle pendant une nuit et récupérer la base libre.

7. Procédé de préparation de la 2'-N-(n-butyl)apramycine suivant la revendication 1, caractérisé en ce qu'il consiste à faire réagir l'apramycine avec l'acétate de nickel à 4 molécules d'eau dans l'eau, diluer dans le diméthylformamide, effectuer un traitement avec le 1-iodo-n-butane pendant 4 jours et récupérer la base libre.

8. Procédé de préparation de la 2'-N-benzylapramycine suivant la revendication 1, caractérisé en ce qu'il consiste à faire réagir l'apramycine avec l'acétate de nickel à 4 molécules d'eau dans l'eau, diluer dans le diméthylformamide, effectuer un traitement avec le bromure de benzyle pendant 19 heures et récupérer une base libre.

9. Procédé de préparation de la 2'-N-allylapramycine suivant la revendication 1, caractérisé en ce qu'il consiste à faire réagir l'apramycine avec l'acétate de nickel à 4 molécules d'eau dans l'eau, diluer avec le diméthylformamide, effectuer un traitement avec le bromure d'allyle pendant 22 heures et récupérer une base libre.